(19) Europäisches Patentamt
European Patent Office
Office européen des brevets

(11) Veröffentlichungsnummer: **0 348 737 B1**

(12) # EUROPÄISCHE PATENTSCHRIFT

(45) Veröffentlichungstag der Patentschrift: **04.08.93**

(51) Int. Cl.$^5$: **C07D 285/12**, C07D 285/00, C07D 285/08, C07D 277/32, C07D 271/10, C07D 273/00, C07D 271/06, C07D 263/34, A01N 43/82, A01N 43/72, A01N 43/76

(21) Anmeldenummer: **89110799.7**

(22) Anmeldetag: **14.06.89**

Die Akte enthält technische Angaben, die nach dem Eingang der Anmeldung eingereicht wurden und die nicht in dieser Patentschrift enthalten sind.

(54) **Heteroaryloxyessigsäure-N-isopropylanilide.**

(30) Priorität: **27.06.88 DE 3821600**

(43) Veröffentlichungstag der Anmeldung:
**03.01.90 Patentblatt 90/01**

(45) Bekanntmachung des Hinweises auf die Patenterteilung:
**04.08.93 Patentblatt 93/31**

(84) Benannte Vertragsstaaten:
**BE CH DE ES FR GB IT LI NL SE**

(56) Entgegenhaltungen:
**EP-A- 0 018 497**
**EP-A- 0 037 526**
**EP-A- 0 094 541**
**EP-A- 0 100 038**
**EP-A- 0 100 044**
**EP-A- 0 100 045**
**EP-A- 0 148 501**
**EP-A- 0 192 117**
**EP-A- 0 195 237**
**EP-A- 0 217 496**
**EP-A- 0 271 975**
**EP-A- 0 300 344**
**DE-A- 3 038 635**

(73) Patentinhaber: **BAYER AG**
**W-5090 Leverkusen 1 Bayerwerk(DE)**

(72) Erfinder: **Förster, Heinz, Dr.**
**Am Eckbusch 47**
**W-5600 Wuppertal 1(DE)**
Erfinder: **Andree, Roland, Dr.**
**Schillerstrasse 17**
**W-4018 Langenfeld(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**
Erfinder: **Strang, Harry, Dr.**
**Unterdorfstrasse 6A**
**W-4000 Düsseldorf 31(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

Erfinder: **Beck, Gunther, Dr.**
**Am Mittelberg 19**
**W-5090 Leverkusen 1(DE)**
Erfinder: **Kysela, Ernst, Dr.**
**Virchowstrasse 14**
**W-5060 Bergisch Gladbach 1(DE)**

## Beschreibung

Die Erfindung betrifft neue Heteroaryloxyessigsäure-N-isopropyl-anilide, ein Verfahren zu ihrer Herstellung und ihre Verwendung als Herbizide.

Es ist bekannt, daß bestimmte Heteroaryloxyessigsäure-anilide, wie z. B. 2-(5-Chlor-1,3,4-thiadiazol-2-yl-oxy)-essigsäure-N-methyl-anilid, herbizide Eigenschaften aufweisen (vgl. EP-A 192 117). Die Nutzpflanzenverträglichkeit dieser bekannten Verbindung ist jedoch nicht immer ganz befriedigend. Vorbeschrieben ist ferner die Verbindung (5-Trifluormethyl-1,3,4-thiadiazol-2-yl)-oxyessigsäure-N-isopropyl-anilid (DE-A-32 18 482; EP-A-94 541; US-A-4,585,471).

Es wurden nun neue Heteroaryloxyessigsäure-N-isopropylanilide der allgemeinen Formel (I) gefunden,

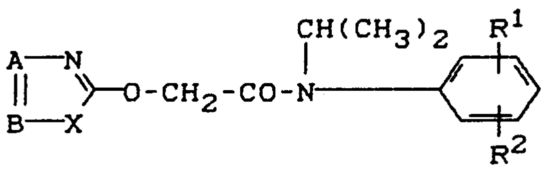

(I)

in welcher

- A für Stickstoff oder die Gruppierung C-$R^3$ steht, wobei
- $R^3$ für Fluor, Chlor, Brom, Cyano, für gegebenenfalls durch Fluor und/oder Chlor substituiertes $C_1$-$C_4$-Alkyl, für gegebenenfalls durch Fluor und/oder Chlor substituiertes $C_1$-$C_4$-Alkoxy, für gegebenenfalls durch Fluor und/oder Chlor substituiertes $C_1$-$C_4$-Alkylthio, für $C_1$-$C_4$-Alkylsulfinyl oder $C_1$-$C_4$-Alkylsulfonyl steht,
- B für Stickstoff oder die Gruppierung C-$R^4$ steht, wobei
- $R^4$ für Fluor, Chlor, Cyano, für gegebenenfalls durch Fluor und/oder Chlor substituiertes $C_1$-$C_4$-Alkyl, für gegebenenfalls durch Fluor und/oder Chlor substituiertes $C_1$-$C_4$-Alkoxy, für gegebenenfalls durch Fluor und/oder Chlor substituiertes $C_1$-$C_4$-Alkylthio, für gegebenenfalls durch Fluor und/oder Chlor substituiertes $C_1$-$C_4$-Alkylsulfinyl, für gegebenenfalls durch Fluor und/oder Chlor substituiertes $C_1$-$C_4$-Alkylsulfonyl, für gegebenenfalls durch Fluor, Chlor, Brom, $C_1$-$C_4$-Alkyl, Trifluormethyl, $C_1$-$C_4$-Alkoxy oder Trifluormethoxy substituiertes Phenyl oder die Gruppierung -$CY_2$-Z-$R^5$ steht, wobei
- $R^5$ für gegebenenfalls durch Fluor und/oder Chlor, $C_1$-$C_4$-Alkoxy oder $C_1$-$C_4$-Alkylthio substituiertes $C_1$-$C_4$-Alkyl oder für gegebenenfalls durch Fluor, Chlor, Brom, $C_1$-$C_4$-Alkyl, Trifluormethyl, $C_1$-$C_4$-Alkoxy, Trifluormethoxy, $C_1$-$C_4$-Alkylthio oder Trifluormethylthio substituiertes Phenyl steht,
- Y für Wasserstoff, Fluor oder Chlor steht,
- Z für Sauerstoff, Schwefel, SO oder $SO_2$ steht,
- $R^1$ für Wasserstoff, Fluor, Chlor, Brom, Cyano, Nitro, für gegebenenfalls durch Fluor und/oder Chlor substituiertes $C_1$-$C_4$-Alkyl, für gegebenenfalls durch Fluor und/oder Chlor substituiertes $C_1$-$C_4$-Alkoxy oder für gegebenenfalls durch Fluor und/oder Chlor substituiertes $C_1$-$C_4$-Alkylthio steht,
- $R^2$ für Wasserstoff, Fluor, Chlor, Brom, Methyl, Trifluormethyl oder Methoxy steht und
- X für Sauerstoff oder Schwefel steht,

ausgenommen die Verbindungen (5-Trifluormethyl-1,3,4-thiadiazol-2-yl)-oxyessigsäure-N-isopropyl-anilid,
(3-Trichlormethyl-1,2,4-thiadiazol-5-yl)-oxy-essigsäure-N-isopropyl-anilid,
(3-Chlor-1,2,4-thiadiazol-5-yl)-oxyessigsäure-N-isopropyl-anilid,
(3-Chlor-1,2,4-thiadiazol-5-yl)-oxyessigsäure-N-isopropyl-N-(3-chorphenyl)-amid,
(3-Chlor-1,2,4-thiadiazol-5-yl)-oxyessigsäure-N-isopropyl-N-(4-chlorphenyl)-amid,
(3-Chlor-1,2,4-thiadiazol-5-yl)-oxyessigsäure-N-isopropyl-N-(2,4-dichlorphenyl)-amid und
(3-Chlor-1,2,4-thiadiazol-5-yl)-oxyessigsäure-N-isopropyl-N-(2-chlorphenyl)-amid.

Weiterhin wurde gefunden, daß man die neuen Heteroaryloxyessigsäure-N-isopropyl-anilide der allgemeinen Formel (I) erhält, wenn man Heteroarene der allgemeinen Formel (II)

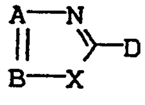

(II)

in welcher

A, B und X die oben angegebene Bedeutung haben und

D für eine nucleofuge Abgangsgruppe steht,

mit Hydroxyessigsäure-N-isopropyl-aniliden der allgemeinen Formel (III)

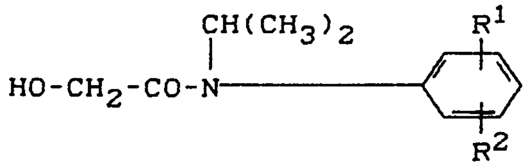

(III)

in welcher

$R^1$ und $R^2$ die oben angegebenen Bedeutungen haben,

gegebenenfalls in Gegenwart eines Verdünnungsmittels, gegebenenfalls in Gegenwart eines Säurebindemittels und gegebenenfalls in Gegenwart eines Katalysators umsetzt.

Schließlich wurde gefunden, daß die neuen Heteroaryloxyessigsäure-N-isopropyl-anilide der allgemeinen Formel (I) interessante herbizide Eigenschaften besitzen.

Überraschenderweise zeigen die neuen Heteroaryloxyessigsäure-N-isopropyl-anilide der allgemeinen Formel (I) bei starker Herbizidwirkung wesentlich bessere selektive Eigenschaften als das bekannte 2-(5-Chlor-1,3,4-thiadiazol-2-yl-oxy)-essigsäure-N-methyl-anilid.

Eine besonders bevorzugte Gruppe von Verbindungen der Formel (I) sind diejenigen der Formel (Ia)

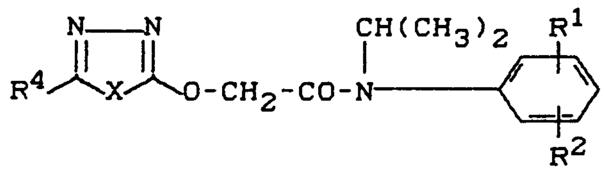

(Ia)

in welcher

$R^1$ für Wasserstoff, Fluor, Chlor, Methyl, Trifluormethyl oder Methoxy steht,

$R^2$ für Wasserstoff steht,

$R^4$ für Chlor, Trifluormethyl, Chlordifluormethyl, Fluordichlormethyl, Dichlormethyl, Trichlormethyl, Pentafluorethyl, Heptafluorpropyl, Methylthio, Ethylthio, Propylthio, Methylsulfinyl, Ethylsulfinyl, Propylsulfinyl, Methylsulfonyl, Ethylsulfonyl, Propylsulfonyl oder Phenyl steht und

X für Sauerstoff oder Schwefel steht,

ausgenommen die Verbindung (5-Trifluormethyl-1,3,4-thiadiazol-2-yl)-oxyessigsäure-N-isopropyl-anilid.

Eine weitere besonders bevorzugte Gruppe von Verbindungen der Formel (I) sind diejenigen der Formel (Ib)

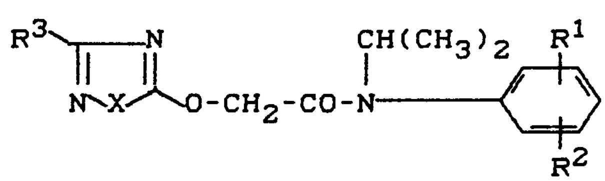

(Ib)

in welcher

$R^1$ für Wasserstoff, Fluor, Chlor, Methyl, Trifluormethyl oder Methoxy steht,

$R^2$ für Wasserstoff steht,

$R^3$ für Chlor, Difluormethyl, Trifluormethyl, Chlordifluormethyl, Fluordichlormethyl, Dichlormethyl, Methyl, Ethyl, Propyl, Isopropyl, Methylthio, Ethylthio, Propylthio, Methylsulfonyl, Ethylsulfonyl, Propylsulfonyl, Methylsulfinyl, Ethylsulfinyl oder Propylsulfinyl steht und

X für Sauerstoff oder Schwefel steht,

ausgenommen die Verbindungen
(3-Chlor-1,2,4-thiadiazol-5-yl)-oxyessigsäure-N-isopropyl-anilid,
(3-Chlor-1,2,4-thiadiazol-5-yl)-oxyessigsäure-N-isopropyl-N-(3-chlorphenyl)-amid,
(3-Chlor-1,2,4-thiadiazol-5-yl)-oxyessigsäure-N-isopropyl-N-(4-chlorphenyl)-amid und
(3-Chlor-1,2,4-thiadiazol-5-yl)-oxyessigsäure-N-isopropyl-N-(2-chlorphenyl)-amid.

Eine dritte besonders bevorzugte Gruppe von Verbindungen der Formel (I) sind diejenigen der Formel (Ic)

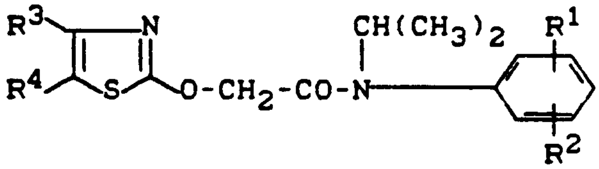

(Ic)

in welcher

$R^1$ für Wasserstoff, Fluor, Chlor, Methyl, Trifluormethyl oder Methoxy steht,

$R^2$ für Wasserstoff steht,

$R^3$ für Chlor, Fluor, Methyl, Difluormethyl, Dichlormethyl, Chlordifluormethyl, Fluordichlormethyl, Trichlormethyl oder Trifluormethyl steht und

$R^4$ für Chlor, Cyano, Difluormethyl, Dichlormethyl, Chlordifluormethyl, Fluordichlormethyl, Trichlormethyl oder Trifluormethyl steht.

Beispiele für die Verbindungen der Formel (I) sind in der nachstehenden Tabelle 1 aufgeführt - vgl. auch die Herstellungsbeispiele.

**Tabelle 1:** Beispiele für die Verbindungen der Formel (I)

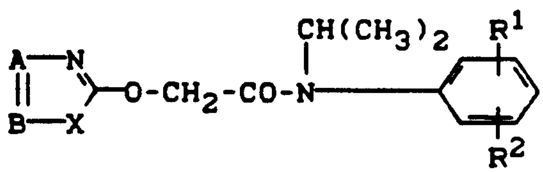

(I)

| A | B | X | $R^1$ | $R^2$ |
|---|---|---|---|---|
| N | C-Cl | S | H | H |
| N | C-Cl | S | 4-F | H |

Tabelle 1 - Fortsetzung

| A | B | X | $R^1$ | $R^2$ |
|---|---|---|---|---|
| N | C-Cl | S | 2-Cl | H |
| N | C-Cl | S | 3-Cl | H |
| N | C-Cl | S | 4-Cl | H |
| N | C-Cl | S | 3-$CH_3$ | H |
| N | C-Cl | S | 4-$CH_3$ | H |
| N | C-Cl | S | 4-$OCH_3$ | H |
| N | C-$CF_3$ | S | 2-F | H |
| N | C-$CF_3$ | S | 4-F | H |
| N | C-$CF_3$ | S | 2-Cl | H |
| N | C-$CF_3$ | S | 3-Cl | H |
| N | C-$CF_3$ | S | 4-Cl | H |
| N | C-$CF_3$ | S | 3-$CH_3$ | H |
| N | C-$CF_3$ | S | 4-$CH_3$ | H |
| N | C-$CF_3$ | S | 4-$OCH_3$ | H |
| N | C-$CF_2Cl$ | S | H | H |
| N | C-$CFCl_2$ | S | H | H |
| N | C-$CHCl_2$ | S | H | H |
| N | C-$CCl_3$ | S | H | H |
| N | C-$C_2F_5$ | S | H | H |
| N | C-$C_3F_7$ | S | H | H |
| N | C-$SCH_3$ | S | H | H |
| N | C-$SOCH_3$ | S | H | H |

Tabelle 1 - Fortsetzung

| A | B | X | $R^1$ | $R^2$ |
|---|---|---|---|---|
| N | $C-SO_2CH_3$ | S | H | H |
| C-Cl | N | O | H | H |
| $C-CHF_2$ | N | S | H | H |
| $C-CF_3$ | N | S | H | H |
| $C-CCl_2F$ | N | S | H | H |
| $C-CF_2Cl$ | N | S | H | H |
| $C-CHCl_2$ | N | S | H | H |
| $C-CH_3$ | N | S | H | H |
| $C-C_2H_5$ | N | S | H | H |
| $C-C_3H_7$ | N | S | H | H |
| $C-CH(CH_3)_2$ | N | S | H | H |
| $C-SCH_3$ | N | S | H | H |
| $C-SC_2H_5$ | N | S | H | H |
| $C-SC_3H_7$ | N | S | H | H |
| $C-SOCH_3$ | N | S | H | H |
| $C-SOC_2H_5$ | N | S | H | H |

Tabelle 1 - Fortsetzung

| A | B | X | $R^1$ | $R^2$ |
|---|---|---|---|---|
| C-$SOC_3H_7$ | N | S | H | H |
| C-$SO_2CH_3$ | N | S | H | H |
| C-$SO_2C_2H_5$ | N | S | H | H |
| C-$SO_2C_3H_7$ | N | S | H | H |
| C-Cl | C-Cl | S | H | H |
| C-Cl | C-Cl | S | 4-F | H |
| C-Cl | C-Cl | S | 2-Cl | H |
| C-Cl | C-Cl | S | 3-Cl | H |
| C-Cl | C-Cl | S | 4-Cl | H |
| C-Cl | C-Cl | S | 3-$CH_3$ | H |
| C-Cl | C-Cl | S | 4-$CH_3$ | H |
| C-Cl | C-Cl | S | 4-$OCH_3$ | H |
| C-Cl | C-CN | S | H | H |
| C-Cl | C-$CHF_2$ | S | H | H |
| C-Cl | C-$CHF_2$ | S | 2-Cl | H |
| C-Cl | C-$CHF_2$ | S | 3-Cl | H |
| C-Cl | C-$CHF_2$ | S | 4-Cl | H |
| C-F | C-$CHF_2$ | S | H | H |
| C-F | C-$CHF_2$ | S | 2-Cl | H |
| C-F | C-$CHF_2$ | S | 3-Cl | H |
| C-F | C-$CHF_2$ | S | 4-Cl | H |

**Tabelle 1** - Fortsetzung

| A | B | X | $R^1$ | $R^2$ |
|---|---|---|---|---|
| $C-CF_3$ | C-CN | S | H | H |
| $C-CF_3$ | C-Cl | S | H | H |
| C-Cl | $C-CF_3$ | S | H | H |
| $C-CH_3$ | C-Cl | S | H | H |
| C-Cl | $C-CF_2Cl$ | S | H | H |
| $C-CH_3$ | C-CN | S | H | H |
| N | $C-CF_3$ | S | $3-CF_3$ | H |

Verwendet man beispielsweise 2,4,5-Trichlorthiazol und Hydroxyessigsäure-N-isopropyl-anilid als Ausgangsstoffe, so läßt sich der Reaktionsablauf beim erfindungsgemäßen Verfahren durch das folgende Formelschema darstellen:

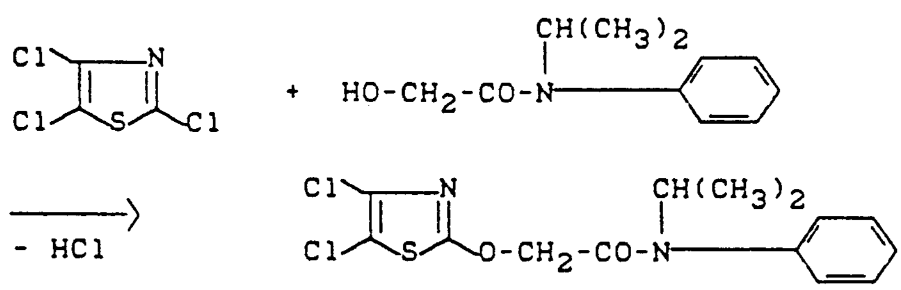


Die beim erfindungsgemäßen Verfahren zur Herstellung von Verbindungen der Formel (I) als Ausgangsstoffe zu verwendenden Heteroarene sind durch die Formel (II) allgemein definiert.

In Formel (II) haben A, B und X diejenigen bevorzugten Bedeutungen, die bei den obigen Formeln (Ia), (Ib) und (Ic) für A, B bzw. X angegeben wurden und D steht vorzugsweise für Halogen oder $C_1$-$C_4$-Alkylsulfonyl, insbesondere für Chlor oder Methylsulfonyl.

Als Beispiele für die Ausgangsstoffe der Formel (II) seien genannt:
2,4,5-Trichlor-thiazol, 2,4-Dichlor-5-cyano-thiazol, 2,4-Dichlor-5-difluormethyl-thiazol, 2,4-Dichlor-5-dichlormethyl-thiazol, 2,4-Dichlor-5-trichlormethyl-thiazol, 2-Chlor-4-fluor-5-difluormethyl-thiazol, 2-Chlor-4-fluor-5-dichlormethyl-thiazol, 2-Chlor-4-fluor-5-trifluormethyl-thiazol, 2-Chlor-4-trifluormethyl-5-cyano-thiazol, 2-Chlor-4-methyl-5-cyano-thiazol, 2,4-Dichlor-5-chlordifluormethyl-thiazol, 2,4-Dichlor-5-fluor-dichlormethyl-thiazol, 2,5-Dichlor-4-methyl-thiazol, 2,5-Dichlor-4-difluormethyl-thiazol, 2,5-Dichlor-4-trichlormethyl-thiazol, 2,5-Dichlor-4-chlordifluormethyl-thiazol, 3,5-Dichlor-1,2,4-thiadiazol, 3-Difluormethyl-5-chlor-1,2,4-thiadiazol, 3-Trifluormethyl-5-chlor-1,2,4-thiadiazol, 3-Chlor-difluormethyl-5-chlor-1,2,4-thiadiazol, 3-Fluordichlormethyl-5-chlor-1,2,4-thiadiazol, 3-Dichlormethyl-5-chlor-1,2,4-thiadiazol, 3-Trichlormethyl-5-chlor-1,2,4-thiadiazol, 3-Methyl-5-chlor-1,2,4-thiadiazol, 3-Methylthio-5-chlor-1,2,4-thiadiazol, 3-Methylsulfinyl-5-chlor-1,2,4-thiadiazol, 3-Methylsulfonyl-5-chlor-1,2,4-thiadiazol, 2,5-Dichlor-1,3,4-thiadiazol, 2-Chlor-5-methylsulfonyl-1,3,4-thiadiazol, 2-Chlor-5-trifluormethyl-1,3,4-thiadiazol, 2-Methylsulfonyl-5-trifluormethyl-1,3,4-thiadiazol, 2-Chlor-5-chlordifluormethyl-1,3,4-thiadiazol, 2-Chlor-5-fluordichlormethyl-1,3,4-thiadiazol, 2-Chlor-5-dichlormethyl-1,3,4-thiadiazol, 2-Chlor-5-trichlormethyl-1,3,4-thiadiazol, 2-Chlor-5-pentafluorethyl-1,3,4-thiadiazol, 2-Chlor-5-heptafluorpropyl-1,3,4-thiadiazol, 2-Chlor-5-methylthio-1,3,4-thiadiazol und 2-Chlor-5-methyl-sulfinyl-1,3,4-thiadiazol.

Die Ausgangsstoffe der Formel (II) sind bekannt und/oder können nach an sich bekannten Verfahren hergestellt werden (vgl. US-P 4 645 525 und dort zit. Lit.; J. Heterocycl. Chem. 11 (1974), 343 - 345; J. Org. Chem. 27 (1962), 2589 - 2592; DE-OS 34 22 861).

Die beim erfindungsgemäßen Verfahren weiter als Ausgangsstoffe zu verwendenden Hydroxyessigsäure-N-isopropyl-anilide sind durch die Formel (III) allgemein definiert.

In der Formel (III) haben $R^1$ und $R^2$ diejenigen bevorzugten Bedeutungen, die bei den obigen Formeln (Ia), (Ib) und (Ic) für $R^1$ bzw. $R^2$ angegeben wurden.

Als Beispiele für die Ausgangsstoffe der Formel (III) seien genannt:
Hydroxyessigsäure-N-isopropyl-anilid, -2-fluor-anilid, -3-fluor-anilid, -4-fluor-anilid, -2-chlor-anilid, -3-chlor-anilid, -4-chlor-anilid, -3-methyl-anilid, -4-methyl-anilid, -3-methoxy-anilid, -4-methoxy-anilid, -3-trifluormethyl-anilid und -4-trifluormethyl-anilid.

Die Hydroxyessigsäure-N-isopropyl-anilide der Formel (III) sind bekannt und/oder können nach an sich bekannten Verfahren hergestellt werden (vgl. US-P 4 509 971 und US-P 4 645 525; ferner US-P 4 334 073, DE-A 3 038 598, DE-A 3 038 636, EP-A 37 526).

Das erfindungsgemäße Verfahren zur Herstellung der neuen Heteroaryloxyessigsäure-N-isopropylanilide der Formel (I) wird vorzugsweise unter Verwendung von Verdünnungsmitteln duchgeführt. Hierzu gehören vorzugsweise Kohlenwasserstoffe, wie z. B. Toluol, Xylol oder Cyclohexan, Halogenkohlenwasserstoffe, wie z. B. Methylenchlorid, Ethylenchlorid, Chloroform oder Chlorbenzol, Ether, wie z. B. Diethylether, Dipropylether, Diisopropylether, Dibutylether, Diisobutylether, Glycoldimethylether, Tetrahydrofuran und Dioxan, Alkohole, wie z. B. Methanol, Ethanol, Propanol, Isopropanol oder Butanol, Ketone, wie z. B. Aceton, Methylethylketon, Methylisopropylketon und Methylisobutylketon, Ester, wie z. B. Essigsäuremethylester und Essigsäureethylester, Amide, wie z. B. Dimethylformamid, Dimethylacetamid und N-Methyl-pyrrolidon, Nitrile, wie z. B. Acetonitril und Propionitril, Sulfoxide, wie z. B. Dimethylsulfoxid sowie Wasser oder wäßrige Salzlösungen.

Als Salze verwendet man hierbei vorzugsweise Chloride oder Sulfate von Alkali- oder Erdalkalimetallen, wie beispielsweise Natriumchlorid, Kaliumchlorid oder Calciumchlorid. Besonders bevorzugt ist Natriumchlorid.

Das erfindungsgemäße Verfahren wird vorteilhaft unter Verwendung von Säurebindemitteln durchgeführt. Als solche werden vorzugsweise stark basische Alkali- und Erdalkalimetallverbindungen, beispielsweise Oxide, wie z. B. Natrium-, Kalium-, Magnesium- und Calciumoxid, Hydroxide, wie z. B. Natrium-, Kalium-, Magnesium- und Calciumhydroxid und/oder Carbonate, wie z. B. Natrium-, Kalium-, Magnesium- und Calciumcarbonat verwendet.

Der Zusatz von 0,01 bis 10 Gew.-% (bezogen auf eingesetztes Glycolsäureamid der Formel (III)) eines Phasentransferkatalysators mag sich in einigen Fällen als vorteilhaft erweisen. Als Beispiele für solche Katalysatoren seien genannt:
Tetrabutylammoniumchlorid, Tetrabutylammoniumbromid, Tributyl-methylphosphoniumbromid, Trimethyl-$C_{13}/C_{15}$-alkyl-ammoniumchlorid, Dibenzyl-dimethyl-ammonium-methylsulfat, Dimethyl-$C_{12}/C_{14}$-alkyl-benzyl-ammoniumchlorid, Tetrabutylammoniumhydroxid, 18-Krone-6, Triethylbenzylammoniumchlorid, Trimethylbenzylammoniumchlorid, Tetraethylammoniumbromid.

Die Reaktionstemperaturen können bei dem erfindungsgemäßen Verfahren in einem größeren Bereich variiert werden. Im allgemeinen arbeitet man bei Temperaturen zwischen -50 °C und +110 °C, vorzugsweise bei Temperaturen zwischen -20 °C und +100 °C.

Das erfindungsgemäße Verfahren wird im allgemeinen bei Normaldruck durchgeführt, es kann aber auch bei erhöhtem oder vermindertem Druck, etwa zwischen 0,1 und 10 bar, durchgeführt werden.

Zur Durchführung des erfindungsgemäßen Verfahrens setzt man je Mol Heteroaren der Formel (II) im allgemeinen 0,5 bis 5 Mol, vorzugsweise 0,8 bis 1,5 Mol, Hydroxyessigsäure-N-isopropyl-anilid der Formel (III) ein. Die Reaktionskomponenten können in beliebiger Reihenfolge zusammengegeben werden. Man rührt jeweils das Reaktionsgemisch bis zum Ende der Umsetzung und arbeitet nach üblichen Methoden auf.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica,

Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich inbesondere zur selektiven Bekämpfung von monokotylen Unkräutern in monokotylen und dikotylen Kulturen, vor allem im Vorauflauf-Verfahren. Sie zeichnen sich besonders durch gute Verträglichkeit in Gerste, Weizen, Mais, Reis, Sonnenblumen und Soja aus.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, als flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2-Chlor-4-ethylamino-6-isopropylamino-1,3,5-triazin (ATRAZIN); 2-[[[[[(4,6-Dimethoxypyrimidin-2-yl)-amino]-carbonyl]-amino]-sulfonyl]-methyl]-benzoesäuremethylester (BENSULFURON); 5-Amino-4-chlor-2-phenyl-2,3-dihydro-3-oxy-pyridazin (CHLORIDAZON); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); 2-Chlor-4-ethylamino-6-(3-cyanopropylamino)-1,3,5-triazin (CYANAZIN); 4-Amino-6-t-butyl-3-ethylthio-1,2,4-triazin-5(4H)-on (ETHIOZIN); 1-Methyl-3-phenyl-5-(3-trifluormethylphenyl)-4-pyridon (FLURIDONE); 5-(2-Chlor-4-trifluormethyl-phenoxy)-N-methylsulfonyl-2-nitrobenzamid (FOMESAFEN); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); N-Methyl-2-(1,3-benzthiazol-2-yloxy)-acetanilid (MEFENACET); 2-{-[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäuremethylester (THIAMETURON) und 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

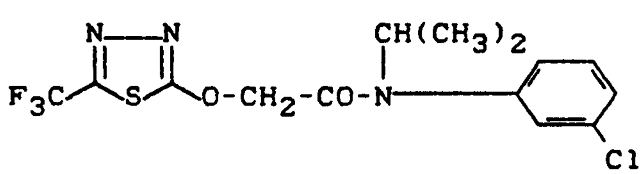

11,8 g (0,05 Mol) 2-Methylsulfonyl-5-trifluormethyl-1,3,4-thiadiazol werden zusammen mit 11,4 g (0,05 Mol) Hydroxyessigsäure-N-isopropyl-(3-chlor-anilid) in 100 ml Aceton gelöst. Bei -20 °C wird eine Lösung aus 2,4 g Natriumhydroxidpulver und 9 ml Wasser langsam zugetropft. Dann läßt man 3 Stunden bei -20 °C nachrühren und gießt das Reaktionsgemisch dann auf Wasser. Das kristalline Produkt wird durch Absaugen isoliert.

Man erhält 16,1 g (85 % der Theorie) (5-Trifluormethyl-1,3,4-thiadiazol-2-yl)-oxyessigsäure-N-isopropyl-(3-chlor-anilid) vom Brechungsindex $n_D^{20}$ : 1,5170.

Analog Beispiel 1 und entsprechend der allgemeinen Beschreibung des erfindungsgemäßen Herstellungsverfahrens können auch die in der nachstehenden Tabelle 2 aufgeführten Verbindungen der Formel (I) hergestellt werden:

Tabelle 2: Herstellungsbeispiele für die Verbindungen der Formel (I)

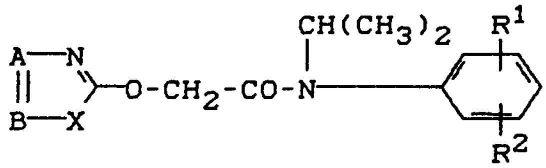

(I)

| Bsp.-Nr. | A | B | X | $R^1$ | $R^2$ | Schmelzpunkt (°C)/ Brechungsindex |
|---|---|---|---|---|---|---|
| 2 | N | C-Cl | S | H | H | 60 |
| 3 | N | $C-CF_3$ | S | $4-OCH_3$ | H | $n_D^{20}$ : 1,5165 |
| 4 | N | $C-CF_3$ | S | $4-CF_3$ | H | |
| 5 | N | $C-CF_3$ | S | 4-Cl | H | 76 |
| 6 | N | $C-CF_3$ | S | 2-Cl | H | 81 |
| 7 | N | C-Cl | S | 3-Cl | H | 71 |
| 8 | N | C-Cl | S | 4-Cl | H | 88 |
| 9 | N | C-Cl | S | 2-Cl | H | $n_D^{20}$ : 1,5645 |
| 10 | $C-CF_2Cl$ | N | S | H | H | 66 |
| 11 | C-F | $C-CHF_2$ | S | H | H | 97 |
| 12 | N | $C-C_3H_7$ | S | H | H | $n_D^{20}$ : 1,4730 |
| 13 | C-Cl | C-CN | S | H | H | 52 |
| 14 | $C-CF_3$ | C-CN | S | H | H | 91 |
| 15 | $C-CCl_2F$ | N | S | H | H | |
| 16 | C-Cl | N | O | H | H | 62 |

Tabelle 2 - Fortsetzung

| Bsp.-Nr. | A | B | X | $R^1$ | $R^2$ | Schmelzpunkt (°C)/ Brechungsindex |
|---|---|---|---|---|---|---|
| 17 | C-Cl | $C-CF_3$ | S | H | H | 110 |
| 18 | $C-SCH_3$ | N | S | H | H | 136 |
| 19 | $C-CH(CH_3)_2$ | N | S | H | H | 45 |
| 20 | $C-CH_3$ | C-CN | S | H | H | 74 |
| 21 | C-Cl | C-Cl | S | H | H | $n_D^{20}$ : 1,5529 |
| 22 | $C-C_3H_7$ | N | S | H | H | 31 |
| 23 | N | $C-SO_2CH_3$ | S | H | H | 133 |
| 24 | $C-SO_2CH_3$ | N | S | H | H | 82 |
| 25 | C-Cl | $C-CHF_2$ | S | H | H | 96 |
| 26 | $C-CF_3$ | N | S | H | H | 63 |
| 27 | $C-SOCH_3$ | N | S | H | H | |
| 28 | N | $C-SC_3H_7$ | S | H | H | 59 |
| 29 | N | C 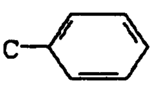 | O | H | H | 157 |
| 30 | N | C 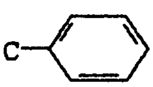 | O | 2-F | H | 172 |
| 31 | N | $C-CF_3$ | S | 3-F | H | 62 |
| 32 | N | $C-CF_3$ | S | 2-F | H | 89 |
| 33 | N | $C-CF_3$ | S | 4-F | H | 60 |
| 34 | N | $C-CF_3$ | S | $3-CH_3$ | H | 86 |

Tabelle 2 - Fortsetzung

| Bsp.-Nr. | A | B | X | $R^1$ | $R^2$ | Schmelzpunkt (°C)/ Brechungsindex |
|---|---|---|---|---|---|---|
| 35 | N | $C-CF_3$ | S | $3-CF_3$ | H | 78 |
| 36 | N | $C-CF_3$ | S | 2-Cl | 4-Cl | 53 |
| 37 | N | $C-CF_3$ | S | $2-OCH_3$ | H | 84 |
| 38 | N | C-Cl | S | $2-OCH_3$ | H | 50 |
| 39 | C-Cl | $C-CHF_2$ | S | $2-OCH_3$ | H | $n_D^{20}$:1.5250 |
| 40 | N | C-Cl | S | $4-OC_2H_5$ | H | 72 |
| 41 | N | $C-CF_3$ | S | $4-OC_2H_5$ | H | $n_D^{20}$:1.5040 |
| 42 | N | C-Cl | S | $4-SCH_3$ | 3-Cl | 134 |
| 43 | N | $C-CF_3$ | S | $4-SCH_3$ | 3-Cl | $n_D^{20}$:1.5475 |
| 44 | N | C-Cl | S | $4-OCH_3$ | H | 88 |
| 45 | N | $C-CF_3$ | S | $4-OCH_3$ | H | 76 |
| 46 | C-Cl | $C-CHF_2$ | S | $4-OCH_3$ | H | $n_D^{20}$:1.5262 |
| 47 | $C-CHF_2$ | C-Cl | S | H | H | 89 |
| 48 | N | $C-CF_3$ | S | 3-Cl | 5-Cl | 102 |
| 49 | $C-CF_3$ | N | S | $2-OCH_3$ | H | 49 |
| 50 | $C-CF_3$ | N | S | $4-OC_2H_5$ | H | 84 |
| 51 | $C-CF_3$ | N | S | $4-SCH_3$ | 3-Cl | $n_D^{20}$:1.5340 |
| 52 | $C-CF_3$ | N | S | $4-OCH_3$ | H | $n_D^{20}$:1.5105 |
| 53 | N | $C-CF_3$ | S | $3-CH_3$ | $5-CH_3$ | 73 |

Tabelle 2 - Fortsetzung

| Bsp.-Nr. | A | B | X | $R^1$ | $R^2$ | Schmelzpunkt (°C)/ Brechungsindex |
|---|---|---|---|---|---|---|
| 54 | N | $C-CF_3$ | S | $4-OCH_3$ | 3-Cl | 81 |
| 55 | N | $C-CF_3$ | S | 3-Cl | 4-Cl | $n_D^{20}$ :1.5181 |
| 56 | N | $C-CF_3$ | S | $3-CF_3$ | $5-CF_3$ | 39 |
| 57 | $C-CHF_2$ | $C-CF_3$ | S | H | H | 50 |
| 58 | C-Cl | $C-CHF_2$ | S | 2-F | H | 86 |
| 59 | C-Cl | $C-CHF_2$ | S | 4-Cl | H | 66 |
| 60 | C-Cl | $C-CHF_2$ | S | 2-Cl | H | 76 |
| 61 | C-Cl | $C-CHF_2$ | S | 3-Cl | H | 68 |
| 62 | C-Cl | $C-CHF_2$ | S | $3-CH_3$ | H | 75 |
| 63 | C-Cl | $C-CHF_2$ | S | 3-F | H | 87 |
| 64 | C-Cl | $C-CHF_2$ | S | 4-F | H | 67 |
| 65 | C-Cl | $C-CHF_2$ | S | 3-Cl | 4-Cl | 50 |
| 66 | C-Cl | $C-CHF_2$ | S | $4-OCH_3$ | 3-Cl | 65 |
| 67 | C-Cl | $C-CHF_2$ | S | $4-OC_2H_5$ | H | |
| 68 | C-Cl | $C-CHF_2$ | S | $3-CF_3$ | $5-CF_3$ | $n_D^{20}$ :1.4740 |
| 69 | C-Cl | $C-CHF_2$ | S | $2-CH_3$ | 5-Cl | 100 |
| 70 | C-Cl | $C-CHF_2$ | S | $3-CH_3$ | $5-CH_3$ | 65 |
| 71 | C-Cl | $C-CHF_2$ | S | 3-Cl | 5-Cl | 81 |
| 72 | N | C-Cl | S | $3-CH_3$ | $5-CH_3$ | 118 |
| 73 | N | C-Cl | S | 3-Cl | 5-Cl | 102 |

Tabelle 2 - Fortsetzung

| Bsp.-Nr. | A | B | X | $R^1$ | $R^2$ | Schmelzpunkt (°C)/ Brechungsindex |
|---|---|---|---|---|---|---|
| 74 | N | C-Cl | S | 4-$OCH_3$ | 3-Cl | 101 |
| 75 | N | C-Cl | S | 3-$CF_3$ | 5-$CF_3$ | 81 |
| 76 | N | C-Cl | S | 3-Cl | 4-Cl | 64 |
| 77 | N | C-Cl | S | 2-$CH_3$ | 5-Cl | 95 |
| 78 | N | C-$CF_3$ | S | 2-$CH_3$ | 5-Cl | 40 |
| 79 | C-$CF_3$ | N | S | 2-Cl | H | 74 |
| 80 | C-$CF_3$ | N | S | 3-F | H | 49 |
| 81 | C-$CF_3$ | N | S | 3-Cl | H | 58 |
| 82 | C-$CF_3$ | N | S | 4-F | H | 57 |
| 83 | C-$CF_3$ | N | S | 2-F | H | 67 |
| 84 | C-$CF_3$ | N | S | 4-Cl | H | $n_D^{20}$ :1.5165 |
| 85 | C-$CF_3$ | N | S | 3-$CH_3$ | H | $n_D^{20}$ :1.5041 |
| 86 | C-$CF_3$ | N | S | 3-$CH_3$ | 5-$CH_3$ | 54 |
| 87 | C-$CF_3$ | N | S | 3-Cl | 5-Cl | 53 |

Ausgangsstoffe der Formel (III)

Beispiel (III-1)

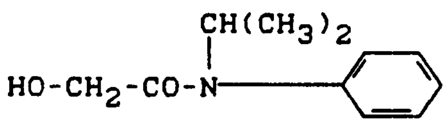

259 g (1,91 Mol) N-Isopropylanilin werden zusammen mit 182 g (2,29 Mol) Pyridin in 2,2 l Toluol gelöst. Bei 0 - 5 °C werden dann 259,7 g (1,91 Mol) Acetoxyacetylchlorid hinzugetropft. Dann läßt man bei 0 - 5 °C 1 Stunde und bei 20 °C 12 Stunden nachrühren. Zur Aufarbeitung wird die Toluolphase mit Wasser und verdünnter Salzsäure ausgeschüttelt, mit Wasser neutralgewaschen, getrocknet und eingeengt. Es bleibt ein

kristalliner beigefarbener Feststoff zurück, der bei 101 °C schmilzt.

Man erhält so 340 g (76 % der Theorie) Acetoxyessigsäure-N-isopropylanilid.

Hiervon werden 334 g (1,42 Mol) in 500 ml Methanol suspendiert. Diese Suspension wird in eine Lösung aus 500 ml Wasser und 63 g Natriumhydroxyd (1,57 Mol) eingetragen. Man läßt 3 Stunden bei 40 °C und dann noch 12 Stunden bei 20 °C rühren. Die Reaktionslösung wird dann mit konzentrierter Salzsäure auf pH 6 eingestellt und am Rotationsverdampfer zur Hälfte eingeengt. Dann gibt man 500 ml Wasser hinzu und extrahiert das Reaktionsprodukt mit 700 ml Chloroform. Die organische Phase wird mit Wasser gewaschen, mit Natriumsulfat getrocknet, filtriert und eingeengt.

Man erhält so 256 g (94 % der Theorie) Hydroxyessigsäure-N-isopropyl-anilid als kristallinen Rückstand vom Schmelzpunkt 39 °C.

Analog Beispiel (III-1) können auch die in der nachfolgenden Tabelle 3 aufgeführten Verbindungen der Formel (III) hergestellt werden:

**Tabelle 3:** Herstellungsbeispiele für Verbindungen der Formel (III)

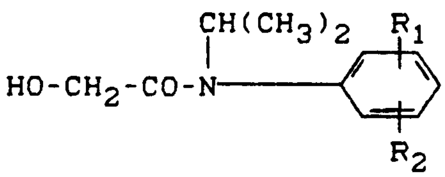

(III)

| Bsp.-Nr. | $R^1$ | $R^2$ | Schmelzpunkt (Siedepunkt) |
|---|---|---|---|
| (III-2) | 4-$OCH_3$ | H | 112° C |
| (III-3) | 3-Cl | H | (Kp: 140° C/1,3 Pa) |
| (III-4) | 4-Cl | H | 75° C |
| (III-5) | 2-Cl | H | 50° C |
| (III-6) | 2-F | H | 51° C |

Anwendungsbeispiel

Im folgenden Anwendungsbeispiel wird die Verbindung nachstehender Formel als Vergleichssubstanz verwendet:

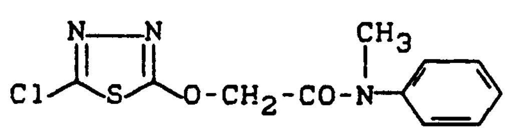

(A)

2-(5-Chlor-1,3,4-thiadiazol-2-yl-oxy)-essigsäure-N-methyl-anilid
(bekannt aus EP-A 192 117).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in Nutzpflanzenselektivität gegenüber dem Stand der Technik zeigen in diesem Test z.B. die Verbindungen gemäß den Herstellungsbeispielen: (1), (5) und (33).

**Patentansprüche**

**Patentansprüche für folgende Vertragsstaaten : BE, CH, DE, FR, GB, IT, LI, NL, SE**

**1.** Heteroaryloxyessigsäure-N-isopropyl-anilide der allgemeinen Formel (I)

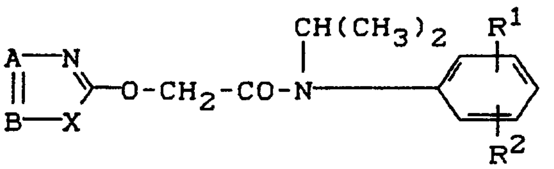

(I)

in welcher

A für Stickstoff oder die Gruppierung C-$R^3$ steht, wobei

$R^3$ für Fluor, Chlor, Brom, Cyano, für gegebenenfalls durch Fluor und/oder Chlor substituiertes $C_1$-$C_4$-Alkyl, für gegebenenfalls durch Fluor und/oder Chlor substituiertes $C_1$-$C_4$-Alkoxy, für gegebenenfalls durch Fluor und/oder Chlor substituiertes $C_1$-$C_4$-Alkylthio, für $C_1$-$C_4$-Alkylsulfinyl oder $C_1$-$C_4$-Alkylsulfonyl steht,

B für Stickstoff oder die Gruppierung C-$R^4$ steht, wobei

$R^4$ für Fluor, Chlor, Cyano, für gegebenenfalls durch Fluor und/oder Chlor substituiertes $C_1$-$C_4$-Alkyl, für gegebenenfalls durch Fluor und/oder Chlor substituiertes $C_1$-$C_4$-Alkoxy, für gegebenenfalls durch Fluor und/oder Chlor substituiertes $C_1$-$C_4$-Alkylthio, für gegebenenfalls durch Fluor und/oder Chlor substituiertes $C_1$-$C_4$-Alkylsulfinyl, für gegebenenfalls durch Fluor und/oder Chlor substituiertes $C_1$-$C_4$-Alkylsulfonyl, für gegebenenfalls durch Fluor, Chlor, Brom, $C_1$-$C_4$-Alkyl, Trifluormethyl, $C_1$-$C_4$-Alkoxy oder Trifluormethoxy substituiertes Phenyl oder die Gruppierung -$CY_2$-Z-$R^5$ steht, wobei

$R^5$ für gegebenenfalls durch Fluor und/oder Chlor, $C_1$-$C_4$-Alkoxy oder $C_1$-$C_4$-Alkylthio substituiertes $C_1$-$C_4$-Alkyl oder für gegebenenfalls durch Fluor, Chlor, Brom, $C_1$-$C_4$-Alkyl, Trifluormethyl, $C_1$-$C_4$-Alkoxy, Trifluormethoxy, $C_1$-$C_4$-Alkylthio oder Trifluormethylthio substituiertes Phenyl steht,

Y für Wasserstoff, Fluor oder Chlor steht,

Z für Sauerstoff, Schwefel, SO oder $SO_2$ steht,

$R^1$ für Wasserstoff, Fluor, Chlor, Brom, Cyano, Nitro, für gegebenenfalls durch Fluor und/oder Chlor substituiertes $C_1$-$C_4$-Alkyl, für gegebenenfalls durch Fluor und/oder Chlor substituiertes $C_1$-$C_4$-Alkoxy oder für gegebenenfalls durch Fluor und/oder Chlor substituiertes $C_1$-$C_4$-

Alkylthio steht,
$R^2$ für Wasserstoff, Fluor, Chlor, Brom, Methyl, Trifluormethyl oder Methoxy steht und
X für Sauerstoff oder Schwefel steht,
ausgenommen die Verbindungen (5-Trifluormethyl-1,3,4-thiadiazol-2-yl)-oxyessigsäure-N-isopropyl-anilid,
(3-Trichlormethyl-1,2,4-thiadiazol-5-yl)-oxyessigsäure-N-isopropyl-anilid,
(3-Chlor-1,2,4-thiadiazol-5-yl)-oxyessigsäure-N-isopropyl-anilid,
(3-Chlor-1,2,4-thiadiazol-5-yl)-oxyessigsäure-N-isopropyl-N-(3-chlorphenyl)-amid,
(3-Chlor-1,2,4-thiadiazol-5-yl)-oxyessigsäure-N-isopropyl-N-(4-chlorphenyl)-amid,
(3-Chlor-1,2,4,-thiadiazol-5-yl)-oxyessigsäure-N-isopropyl-N-(2,4-dichlorphenyl)-amid und
(3-Chlor-1,2,4-thiadiazol-5-yl)-oxyessigsäure-N-isopropyl-N-(2-chlorphenyl)-amid.

**2.** Heteroaryloxyessigsäure-N-isopropyl-anilide der Formel (Ia)

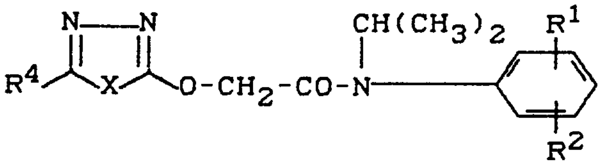
(Ia)

in welcher
$R^1$ für Wasserstoff, Fluor, Chlor, Methyl, Trifluormethyl oder Methoxy steht,
$R^2$ für Wasserstoff steht,
$R^4$ für Chlor, Trifluormethyl, Chlordifluormethyl, Fluordichlormethyl, Dichlormethyl, Trichlormethyl, Pentafluorethyl, Heptafluorpropyl, Methylthio, Ethylthio, Propylthio, Methylsulfinyl, Ethylsulfinyl, Propylsulfinyl, Methylsulfonyl, Ethylsulfonyl, Propylsulfonyl oder Phenyl steht und
X für Sauerstoff oder Schwefel steht,
gemäß Anspruch 1, ausgenommen die Verbindung (5-Trifluormethyl-1,3,4-thiadiazol-2-yl)-oxyessigsäure-N-isopropyl-anilid.

**3.** Heteroaryloxyessigsäure-N-isopropyl-anilide der Formel (Ib)

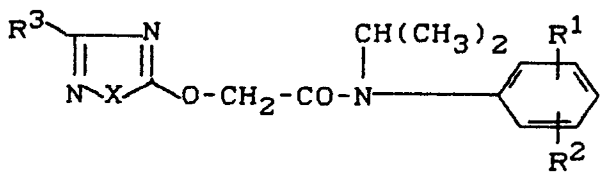
(Ib)

in welcher
$R^1$ für Wasserstoff, Fluor, Chlor, Methyl, Trifluormethyl oder Methoxy steht,
$R^2$ für Wasserstoff steht,
$R^3$ für Chlor, Difluormethyl, Trifluormethyl, Chlordifluormethyl, Fluordichlormethyl, Dichlormethyl, Methyl, Ethyl, Propyl, Isopropyl, Methylthio, Ethylthio, Propylthio, Methylsulfonyl, Ethylsulfonyl, Propylsulfonyl, Methylsulfinyl, Ethylsulfinyl oder Propylsulfinyl steht und
X für Sauerstoff oder Schwefel steht,
gemäß Anspruch 1, ausgenommen die Verbindungen (3-Chlor-1,2,4-thiadiazol-5-yl)-oxyessigsäure-N-isopropyl-anilid,
(3-Chlor-1,2,4-thiadiazol-5-yl)-oxyessigsäure-N-isopropyl-N-(3-chlorphenyl)-amid,
(3-Chlor-1,2,4-thiadiazol-5-yl)-oxyessigsäure-N-isopropyl-N-(4-chlorphenyl)-amid und
(3-Chlor-1,2,4-thiadiazol-5-yl)-oxyessigsäure-N-isopropyl-N-(2-chlorphenyl)-amid.

**4.** Heteroaryloxyessigsäure-N-isopropyl-anilide der Formel (Ic)

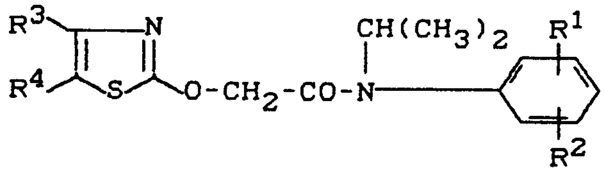

(Ic)

in welcher

$R^1$ für Wasserstoff, Fluor, Chlor, Methyl, Trifluormethyl oder Methoxy steht,

$R^2$ für Wasserstoff steht,

$R^3$ für Chlor, Fluor, Methyl, Difluormethyl, Dichlormethyl, Chlordifluormethyl, Fluordichlormethyl, Trichlormethyl oder Trifluormethyl steht und

$R^4$ für Chlor, Cyano, Difluormethyl, Dichlormethyl, Chlordifluormethyl, Fluordichlormethyl, Trichlormethyl oder Trifluormethyl steht,

gemäß Anspruch 1.

**5.** (5-Trifluormethyl-1,3,4-thiadiazol-5-yl)-oxyessigsäure-N-isopropyl-N-(4-fluorphenyl)-amid der Formel

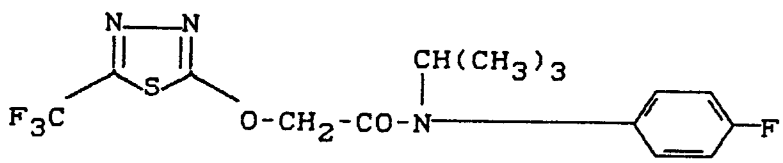

(33)

gemäß Ansprüchen 1 und 2.

**6.** Verfahren zur Herstellung von Heteroaryloxyessigsäure-N-isopropyl-aniliden der allgemeinen Formel (I) gemäß Anspruch 1, dadurch gekennzeichnet, daß man Heteroarene der allgemeinen Formel (II)

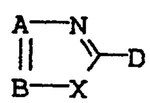

(II)

in welcher

A, B und X die in Anspruch 1 angegebenen Bedeutungen haben und

D für eine nucleofuge Abgangsgruppe steht,

mit Hydroxyessigsäure-N-isopropyl-aniliden der allgemeinen Formel (III)

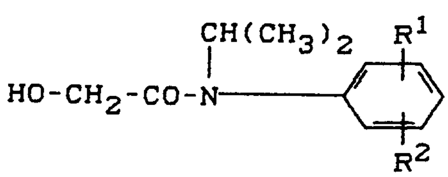

(III)

in welcher

$R^1$ und $R^2$ die in Anspruch 1 angegebenen Bedeutungen haben,

gegebenenfalls in Gegenwart eines Verdünnungsmittels, gegebenenfalls in Gegenwart eines Säurebindemittels und gegebenenfalls in Gegenwart eines Katalysators umsetzt.

**7.** Herbizide Mittel, gekennzeichnet durch einen Gehalt an mindestens einem Heteroaryloxyessigsäure-N-isopropyl-anilid der Formel (I) gemäß Anspruch 1.

**8.** Verfahren zur Bekämpfung von unerwünschtem Pflanzenwachstum, dadurch gekennzeichnet, daß man auf die Pflanzen oder ihren Lebensraum Heteroaryloxyessigsäure-N-isopropyl-anilideder Formel (I) gemäß Anspruch 1 einwirken läßt.

**9.** Verwendung von Heteroaryloxyessigsäure-N-isopropyl-aniliden der Formel (I) gemäß Anspruch 1 zur Bekämpfung von unerwünschtem Pflanzenwachstum.

**10.** Verfahren zur Herstellung von herbiziden Mitteln, dadurch gekennzeichnet, daß man Heteroaryloxyessigsäure-N-isopropyl-anilide der Formel (I) gemäß Anspruch 1 mit Streckmitteln und/oder oberflächenaktiven Mitteln vermischt.

**Patentansprüche für folgenden Vertragsstaat : ES**

**1.** Verfahren zur Herstellung von Heteroaryloxyessigsäure-N-isopropyl-aniliden der allgemeinen Formel (I),

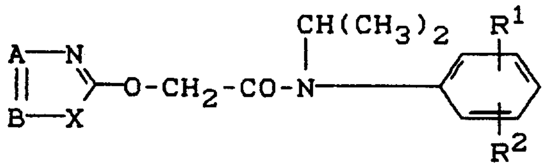

(I)

in welcher

A für Stickstoff oder die Gruppierung C-$R^3$ steht, wobei

$R^3$ für Fluor, Chlor, Brom, Cyano, für gegebenenfalls durch Fluor und/oder Chlor substituiertes $C_1$-$C_4$-Alkyl, für gegebenenfalls durch Fluor und/oder Chlor substituiertes $C_1$-$C_4$-Alkoxy, für gegebenenfalls durch Fluor und/oder Chlor substituiertes $C_1$-$C_4$-Alkylthio, für $C_1$-$C_4$-Alkylsulfinyl oder $C_1$-$C_4$-Alkylsulfonyl steht,

B für Stickstoff oder die Gruppierung C-$R^4$ steht, wobei

$R^4$ für Fluor, Chlor, Cyano, für gegebenenfalls durch Fluor und/oder Chlor substituiertes $C_1$-$C_4$-Alkyl, für gegebenenfalls durch Fluor und/oder Chlor substituiertes $C_1$-$C_4$-Alkoxy, für gegebenenfalls durch Fluor und/oder Chlor substituiertes $C_1$-$C_4$-Alkylthio, für gegebenenfalls durch Fluor und/oder Chlor substituiertes $C_1$-$C_4$-Alkylsulfinyl, für gegebenenfalls durch Fluor und/oder Chlor substituiertes $C_1$-$C_4$-Alkylsulfonyl, für gegebenenfalls durch Fluor, Chlor, Brom, $C_1$-$C_4$-Alkyl, Trifluormethyl, $C_1$-$C_4$-Alkoxy oder Trifluormethoxy substituiertes Phenyl oder die Gruppierung -$CY_2$-Z-$R^5$ steht, wobei

$R^5$ für gegebenenfalls durch Fluor und/oder Chlor, $C_1$-$C_4$-Alkoxy oder $C_1$-$C_4$-Alkylthio substituiertes $C_1$-$C_4$-Alkyl oder für gegebenenfalls durch Fluor, Chlor, Brom, $C_1$-$C_4$-Alkyl, Trifluormethyl, $C_1$-$C_4$-Alkoxy, Trifluormethoxy, $C_1$-$C_4$-Alkylthio oder Trifluormethylthio substituiertes Phenyl steht,

Y für Wasserstoff, Fluor oder Chlor steht,

Z für Sauerstoff, Schwefel, SO oder $SO_2$ steht,

$R^1$ für Wasserstoff, Fluor, Chlor, Brom, Cyano, Nitro, für gegebenenfalls durch Fluor und/oder Chlor substituiertes $C_1$-$C_4$-Alkyl, für gegebenenfalls durch Fluor und/oder Chlor substituiertes $C_1$-$C_4$-Alkoxy oder für gegebenenfalls durch Fluor und/oder Chlor substituiertes $C_1$-$C_4$-Alkylthio steht,

$R^2$ für Wasserstoff, Fluor, Chlor, Brom, Methyl, Trifluormethyl oder Methoxy steht und

X für Sauerstoff oder Schwefel steht,

ausgenommen die Verbindungen (5-Trifluormethyl-1,3,4-thiadiazol-2-yl)-oxyessigsäure-N-isopropyl-anilid,

(3-Trichlormethyl-1,2,4-thiadiazol-5-yl)-oxyessigsäure-N-isopropyl-anilid,
(3-Chlor-1,2,4-thiadiazol-5-yl)-oxyessigsäure-N-isopropyl-anilid,
(3-Chlor-1,2,4-thiadiazol-5-yl)-oxyessigsäure-N-isopropyl-N-(3-chlorphenyl)-amid,
(3-Chlor-1,2,4-thiadiazol-5-yl)-oxyessigsäure-N-isopropyl-N-(4-chlorphenyl)-amid,
(3-Chlor-1,2,4,-thiadiazol-5-yl)-oxyessigsäure-N-isopropyl-N-(2,4-dichlorphenyl)-amid und
(3-Chlor-1,2,4-thiadiazol-5-yl)-oxyessigsäure-N-isopropyl-N-(2-chlorphenyl)-amid,
dadurch gekennzeichnet, daß man Heteroarene der allgemeinen Formel (II),

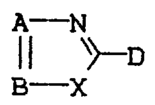

(II)

in welcher

A, B und X die oben bei Formel (I) angegebenen Bedeutungen haben und
D für eine nucleofuge Abgangsgruppe steht,

mit Hydroxyessigsäure-N-isopropyl-aniliden der allgemeinen Formel (III),

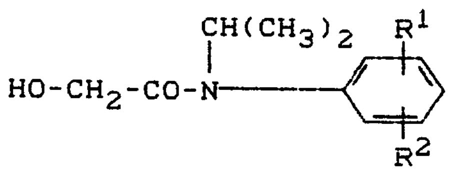

(III)

in welcher

$R^1$ und $R^2$ die oben bei Formel (I) angegebenen Bedeutungen haben,

gegebenenfalls in Gegenwart eines Verdünnungsmittels, gegebenenfalls in Gegenwart eines Säurebindemittels und gegebenenfalls in Gegenwart eines Katalysators umsetzt.

**2.** Verfahren zur Herstellung von Heteroaryloxyessigsäure-N-isopropyl-aniliden der Formel (Ia),

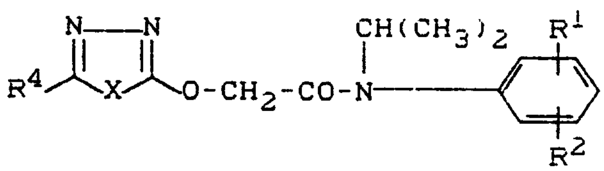

(Ia)

in welcher

$R^1$ für Wasserstoff, Fluor, Chlor, Methyl, Trifluormethyl oder Methoxy steht,
$R^2$ für Wasserstoff steht,
$R^4$ für Chlor, Trifluormethyl, Chlordifluormethyl, Fluordichlormethyl, Dichlormethyl, Trichlormethyl, Pentafluorethyl, Heptafluorpropyl, Methylthio, Ethylthio, Propylthio, Methylsulfinyl, Ethylsulfinyl, Propylsulfinyl, Methylsulfonyl, Ethylsulfonyl, Propylsulfonyl oder Phenyl steht und
X für Sauerstoff oder Schwefel steht,

gemäß Anspruch 1, ausgenommen die Verbindung (5-Trifluormethyl-1,3,4-thiadiazol-2-yl)-oxyessigsäure-N-isopropyl-anilid,
dadurch gekennzeichnet, daß man Heteroarene der allgemeinen Formel (II),

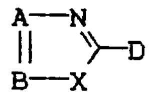 (II)

in welcher

A, B und X die oben bei Formel (Ia) angegebenen Bedeutungen haben und

D für eine nucleofuge Abgangsgruppe steht,

mit Hydroxyessigsäure-N-isopropyl-aniliden der allgemeinen Formel (III),

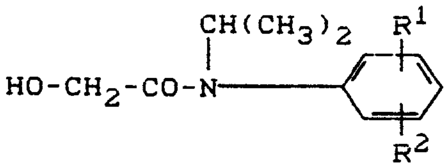 (III)

in welcher

$R^1$ und $R^2$ die oben bei Formel (Ia) angegebenen Bedeutungen haben,

gegebenenfalls in Gegenwart eines Verdünnungsmittels, gegebenenfalls in Gegenwart eines Säurebindemittels und gegebenenfalls in Gegenwart eines Katalysators umsetzt.

**3.** Verfahren zur Herstellung von Heteroaryloxyessigsäure-N-isopropyl-aniliden der Formel (Ib),

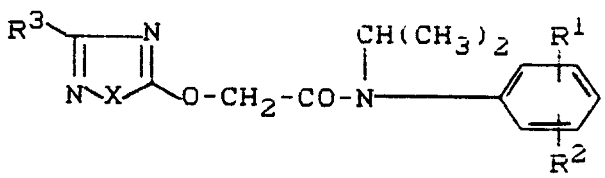 (Ib)

in welcher

$R^1$ für Wasserstoff, Fluor, Chlor, Methyl, Trifluormethyl oder Methoxy steht,

$R^2$ für Wasserstoff steht,

$R^3$ für Chlor, Difluormethyl, Trifluormethyl, Chlordifluormethyl, Fluordichlormethyl, Dichlormethyl, Methyl, Ethyl, Propyl, Isopropyl, Methylthio, Ethylthio, Propylthio, Methylsulfonyl, Ethylsulfonyl, Propylsulfonyl, Methylsulfinyl, Ethylsulfinyl oder Propylsulfinyl steht und

X für Sauerstoff oder Schwefel steht,

gemäß Anspruch 1, ausgenommen die Verbindungen (3-Chlor-1,2,4-thiadiazol-5-yl)-oxyessigsäure-N-isopropyl-anilid,

(3-Chlor-1,2,4-thiadiazol-5-yl)-oxyessigsäure-N-isopropyl-N-(3-chlorphenyl)-amid,

(3-Chlor-1,2,4-thiadiazol-5-yl)-oxyessigsäure-N-isopropyl-N-(4-chlorphenyl)-amid und

(3-Chlor-1,2,4-thiadiazol-5-yl)-oxyessigsäure-N-isopropyl-N-(2-chlorphenyl)-amid,

dadurch gekennzeichnet, daß man Heteroarene der allgemeinen Formel (II),

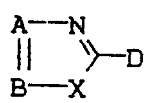 (II)

in welcher

A, B und X die oben bei Formel (Ib) angegebenen Bedeutungen haben und

D für eine nucleofuge Abgangsgruppe steht,

mit Hydroxyessigsäure-N-isopropyl-aniliden der allgemeinen Formel (III),

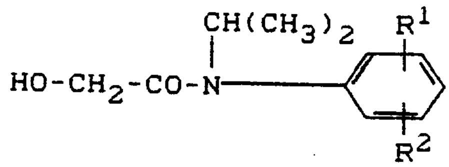

(III)

in welcher

$R^1$ und $R^2$ die oben bei Formel (Ib) angegebenen Bedeutungen haben,

gegebenenfalls in Gegenwart eines Verdünnungsmittels, gegebenenfalls in Gegenwart eines Säurebindemittels und gegebenenfalls in Gegenwart eines Katalysators umsetzt.

**4.** Verfahren zur Herstellung von Heteroaryloxyessigsäure-N-isopropyl-aniliden der Formel (Ic),

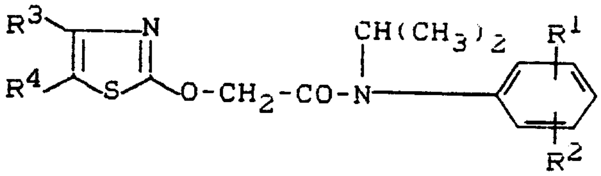

(Ic)

in welcher

- $R^1$ für Wasserstoff, Fluor, Chlor, Methyl, Trifluormethyl oder Methoxy steht,
- $R^2$ für Wasserstoff steht,
- $R^3$ für Chlor, Fluor, Methyl, Difluormethyl, Dichlormethyl, Chlordifluormethyl, Fluordichlormethyl, Trichlormethyl oder Trifluormethyl steht und
- $R^4$ für Chlor, Cyano, Difluormethyl, Dichlormethyl, Chlordifluormethyl, Fluordichlormethyl, Trichlormethyl oder Trifluormethyl steht,

gemäß Anspruch 1,

dadurch gekennzeichnet, daß man Heteroarene der allgemeinen Formel (II),

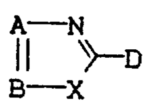

(II)

in welcher

A, B und X die oben bei Formel (Ic) angegebenen Bedeutungen haben und

D für eine nucleofuge Abgangsgruppe steht,

mit Hydroxyessigsäure-N-isopropyl-aniliden der allgemeinen Formel (III),

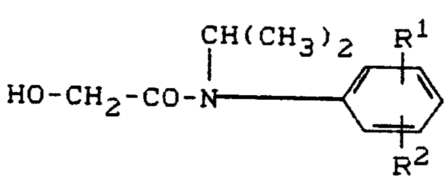

(III)

in welcher

$R^1$ und $R^2$ die oben bei Formel (Ic) angegebenen Bedeutungen haben,

gegebenenfalls in Gegenwart eines Verdünnungsmittels, gegebenenfalls in Gegenwart eines Säurebindemittels und gegebenenfalls in Gegenwart eines Katalysators umsetzt.

5. Verfahren zur Herstellung von (5-Trifluormethyl-1,3,4-thiadiazol-5-yl)-oxyessigsäure-N-isopropyl-N-(4-fluorphenyl)-amid der Formel

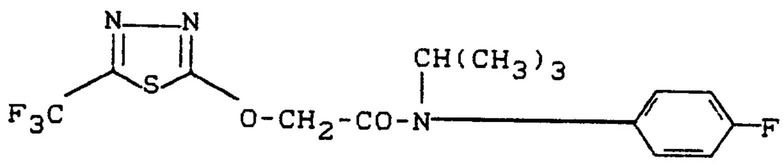

(33)

gemäß Ansprüchen 1 und 2, dadurch gekennzeichnet, daß man ein Heteroaren der allgemeinen Formel (II),

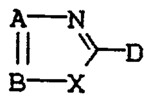

(II)

in welcher
- A für N steht,
- B für $F_3C$-C steht,
- X für S steht und
- D für eine nucleofuge Abgangsgruppe steht,

mit einem Hydroxyessigsäure-N-isopropyl-anilid der allgemeinen Formel (III),

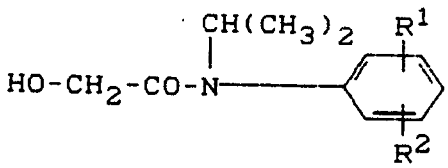

(III)

in welcher
- $R^1$ für 4-F und
- $R^2$ für H steht,

gegebenenfalls in Gegenwart eines Verdünnungsmittels, gegebenenfalls in Gegenwart eines Säurebindemittels und gegebenenfalls in Gegenwart eines Katalysators umsetzt.

6. Herbizide Mittel, gekennzeichnet durch einen Gehalt an mindestens einem Heteroaryloxyessigsäure-N-isopropyl-anilid der Formel (I) gemäß Anspruch 1.

7. Verfahren zur Bekämpfung von unerwünschtem Pflanzenwachstum, dadurch gekennzeichnet, daß man auf die Pflanzen oder ihren Lebensraum Heteroaryloxyessigsäure-N-isopropyl-anilideder Formel (I) gemäß Anspruch 1 einwirken läßt.

8. Verwendung von Heteroaryloxyessigsäure-N-isopropyl-aniliden der Formel (I) gemäß Anspruch 1 zur Bekämpfung von unerwünschtem Pflanzenwachstum.

9. Verfahren zur Herstellung von herbiziden Mitteln, dadurch gekennzeichnet, daß man Heteroaryloxyessigsäure-N-isopropyl-anilide der Formel (I) gemäß Anspruch 1 mit Streckmitteln und/oder oberflächenaktiven Mitteln vermischt.

## Claims

**Claims for the following Contracting States : BE, CH, DE, FR, GB, IT, LI, NL, SE**

**1.** N-Isopropylheteroaryloxyacetanilides of the general formula (I)

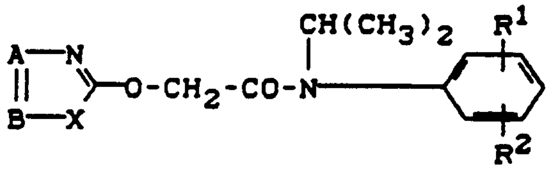

(I)

in which

A represents nitrogen or the group C-$R^3$ where

$R^3$ represents fluorine, chlorine, bromine, cyano, $C_1$-$C_4$-alkyl which is optionally substituted by fluorine and/or chlorine, $C_1$-$C_4$-alkoxy which is optionally substituted by fluorine and/or chlorine, $C_1$-$C_4$-alkylthio which is optionally substituted by fluorine and/or chlorine, $C_1$-$C_4$-alkylsulphinyl or $C_1$-$C_4$-alkylsulphonyl,

B represents nitrogen or the group C-$R^4$ where

$R^4$ represents fluorine, chlorine, cyano, $C_1$-$C_4$-alkyl which is optionally substituted by fluorine and/or chlorine, $C_1$-$C_4$-alkoxy which is optionally substituted by fluorine and/or chlorine, $C_1$-$C_4$-alkylthio which is optionally substituted by fluorine and/or chlorine, $C_1$-$C_4$-alkylsulphinyl which is optionally substituted by fluorine and/or chlorine, $C_1$-$C_4$-alkylsulphonyl which is optionally substituted by fluorine and/or chlorine, phenyl which is optionally substituted by fluorine, chlorine, bromine, $C_1$-$C_4$-alkyl, trifluoromethyl, $C_1$-$C_4$-alkoxy or trifluoromethoxy, or the group $-CY_2-Z-R^5$ where

$R^5$ represents $C_1$-$C_4$-alkyl which is optionally substituted by fluorine and/or chlorine, $C_1$-$C_4$-alkoxy or $C_1$-$C_4$-alkylthio or phenyl which is optionally substituted by fluorine, chlorine, bromine, $C_1$-$C_4$-alkyl, trifluoromethyl, $C_1$-$C_4$-alkoxy, trifluoromethoxy, $C_1$-$C_4$-alkylthio or trifluoromethylthio,

Y represents hydrogen, fluorine or chlorine,

Z represents oxygen, sulphur, SO or $SO_2$,

$R^1$ represents hydrogen, fluorine, chlorine, bromine, cyano, nitro, or $C_1$-$C_4$-alkyl which is optionally substituted by fluorine and/or chlorine, $C_1$-$C_4$-alkoxy which is optionally substituted by fluorine and/or chlorine or $C_1$-$C_4$-alkylthio which is optionally substituted by fluorine and/or chlorine,

$R^2$ represents hydrogen, fluorine, chlorine, bromine, methyl, trifluoromethyl or methoxy, and

X represents oxygen or sulphur,

excluding the compounds N-isopropyl-(5-trifluoromethyl-1,3,4-thiadiazol-2-yl)-oxyacetanilide,
N-isopropyl-(3-trichloromethyl-1,2,4-thiadiazol-5-yl)-oxyacetanilide,
N-isopropyl-(3-chloro-1,2,4-thiadiazol-5-yl)-oxyacetanilide,
N-isopropyl-N-(3-chlorophenyl)-(3-chloro-1,2,4-thiadiazol-5-yl)-oxyacetamide,
N-isopropyl-N-(4-chlorophenyl)-(3-chloro-1,2,4-thiadiazol-5-yl)-oxyacetamide,
N-isopropyl-N-(2,4-dichlorophenyl)-(3-chloro-1,2,4-thiadiazol-5-yl)-oxyacetamide and
N-isopropyl-N-(2-chlorophenyl)-(3-chloro-1,2,4-thiadiazol-5-yl)-oxyacetamide.

**2.** N-Isopropylheteroaryloxyacetanilides of the formula (Ia)

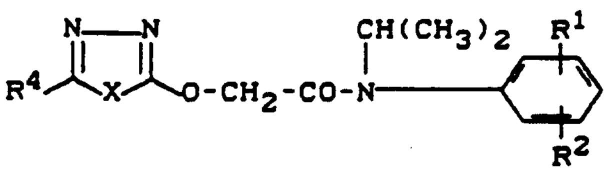

(Ia)

in which

- $R^1$ represents hydrogen, fluorine, chlorine, methyl, trifluoromethyl or methoxy,
- $R^2$ represents hydrogen,
- $R^4$ represents chlorine, trifluoromethyl, chlorodifluoromethyl, fluorodichloromethyl, dichloromethyl, trichloromethyl, pentafluoroethyl, heptafluoropropyl, methylthio, ethylthio, propylthio, methylsulphinyl, ethylsulphinyl, propylsulphinyl, methylsulphonyl, ethylsulphonyl, propylsulphonyl or phenyl and
- X represents oxygen or sulphur,

according to Claim 1, excluding the compound N-isopropyl-(5-trifluoromethyl-1,3,4-thiadiazol-2-yl)-oxyacetanilide.

**3.** N-Isopropylheteroaryloxyacetanilides of the formula (Ib)

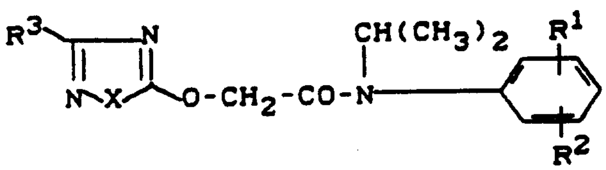

(Ib)

in which

- $R^1$ represents hydrogen, fluorine, chlorine, methyl, trifluoromethyl or methoxy,
- $R^2$ represents hydrogen,
- $R^3$ represents chlorine, difluoromethyl, trifluoromethyl, chlorodifluoromethyl, fluorodichloromethyl, dichloromethyl, methyl, ethyl, propyl, isopropyl, methylthio, ethylthio, propylthio, methylsulphonyl, ethylsulphonyl, propylsulphonyl, methylsulphinyl, ethylsulphinyl or propylsulphinyl and
- X represents oxygen or sulphur,

according to Claim 1, excluding the compounds N-isopropyl-(3-chloro-1,2,4-thiadiazol-5-yl)-oxyacetanilide,
N-isopropyl-N-(3-chlorophenyl)-(3-chloro-1,2,4-thiadiazol-5-yl)-oxyacetamide,
N-isopropyl-N-(4-chlorophenyl)-(3-chloro-1,2,4-thiadiazol-5-yl)-oxyacetamide, and
N-isopropyl-N-(2-chlorophenyl)-(3-chloro-1,2,4-thiadiazol-5-yl)-oxyacetamide.

**4.** N-Isopropylheteroaryloxyacetanilides of the formula (Ic)

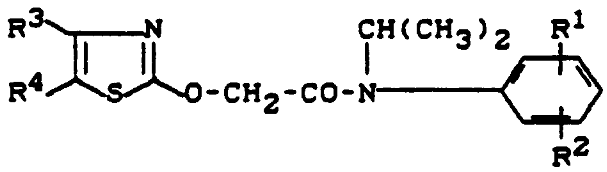

(Ic)

in which

- $R^1$ represents hydrogen, fluorine, chlorine, methyl, trifluoromethyl or methoxy,
- $R^2$ represents hydrogen,
- $R^3$ represents chlorine, fluorine, methyl, difluoromethyl, dichloromethyl, chlorodifluoromethyl, fluorodichloromethyl, trichloromethyl or trifluoromethyl and
- $R^4$ represents chlorine, cyano, difluoromethyl, dichloromethyl, chlorodifluoromethyl, fluorodichloromethyl, trichloromethyl or trifluoromethyl

according to Claim 1.

5. N-isopropyl-N-(4-fluorophenyl)-(5-trifluoromethyl-1,3,4-thiadiazol-5-yl)-oxyacetamide of the formula

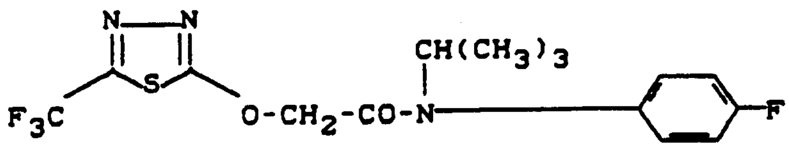

(33)

according to Claims 1 and 2.

6. Process for the preparation of N-isopropylheteroaryloxyacetanilides of the general formula (I) according to Claim 1, characterised in that heteroarenes of the general formula (II)

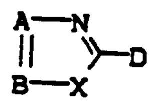

(II)

in which
A, B and X have the meanings given in Claim 1 and
D represents for a nucleofugic leaving group
are reacted with N-isopropylhydroxyacetamides of the general formula (III)

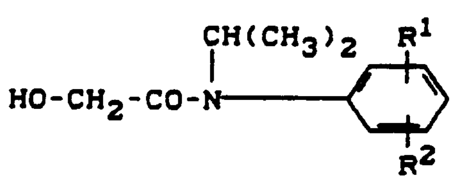

(III)

in which
$R^1$ and $R^2$ have the meanings given in Claim 1,
if appropriate in the presence of a diluent, if appropriate in the presence of an acid-binding agent and if appropriate in the presence of a catalyst.

7. Herbicidal agents, characterised in that they contain at least one N-isopropylheteroaryloxyacetanilide of the formula (I) according to Claim 1.

8. Method of combating undesired plant growth, characterised in that the N-isopropylheteroaryloxyacetanilides of the formula (I) according to Claim 1 are allowed to act on the plants or their environment.

9. Use of N-isopropylheteroaryloxyacetanilides of the formula (I) according to Claim 1 for combating undesired plant growth.

10. Process for the preparation of herbicidal agents, characterised in that N-isopropylheteroaryloxyacetanilides of the formula (I) according to Claim 1 are mixed with extenders and/or surface-active agents.

**Claims for the following Contracting State : ES**

**1.** Process for the preparation of N-isopropylheteroarylacetanilides of the general formula (I)

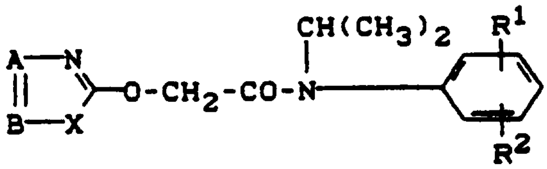

(I)

in which

A represents nitrogen or the group C-$R^3$ where

$R^3$ represents fluorine, chlorine, bromine, cyano, $C_1$-$C_4$-alkyl which is optionally substituted by fluorine and/or chlorine, $C_1$-$C_4$-alkoxy which is optionally substituted by fluorine and/or chlorine, $C_1$-$C_4$-alkylthio which is optionally substituted by fluorine and/or chlorine, $C_1$-$C_4$-alkylsulphinyl or $C_1$-$C_4$-alkylsulphonyl,

B represents nitrogen or the group C-$R^4$ where

$R^4$ represents fluorine, chlorine, cyano, $C_1$-$C_4$-alkyl which is optionally substituted by fluorine and/or chlorine, $C_1$-$C_4$-alkoxy which is optionally substituted by fluorine and/or chlorine, $C_1$-$C_4$-alkylthio which is optionally substituted by fluorine and/or chlorine, $C_1$-$C_4$-alkylsulphinyl which is optionally substituted by fluorine and/or chlorine, $C_1$-$C_4$-alkylsulphonyl which is optionally substituted by fluorine and/or chlorine, phenyl which is optionally substituted by fluorine, chlorine, bromine, $C_1$-$C_4$-alkyl, trifluoromethyl, $C_1$-$C_4$-alkoxy or trifluoromethoxy, or the group -$CY_2$-Z-$R^5$ where

$R^5$ represents $C_1$-$C_4$-alkyl which is optionally substituted by fluorine and/or chlorine, $C_1$-$C_4$-alkoxy or $C_1$-$C_4$-alkylthio, or phenyl which is optionally substituted by fluorine, chlorine, bromine, $C_1$-$C_4$-alkyl, trifluoromethyl, $C_1$-$C_4$-alkoxy, trifluoromethoxy, $C_1$-$C_4$-alkylthio or trifluoromethylthio,

Y represents hydrogen, fluorine or chlorine,

Z represents oxygen, sulphur, SO or $SO_2$,

$R^1$ represents hydrogen, fluorine, chlorine, bromine, cyano, nitro, $C_1$-$C_4$-alkyl which is optionally substituted by fluorine and/or chlorine, $C_1$-$C_4$-alkoxy which is optionally substituted by fluorine and/or chlorine, or $C_1$-$C_4$-alkylthio which is optionally substituted by fluorine and/or chlorine,

$R^2$ represents hydrogen, fluorine, chlorine, bromine, methyl, trifluoromethyl or methoxy and

X represents oxygen or sulphur,

excluding the compounds N-isopropyl-(5-trifluoromethyl-1,3,4-thiadiazol-2-yl)-oxyacetanilide,
N-isopropyl-(3-trichloromethyl-1,2,4-thiadiazol-5-yl)-oxyacetanilide,
N-isopropyl-(3-chloro-1,2,4-thiadiazol-5-yl)-oxyacetanilide,
N-isopropyl-N-(3-chlorophenyl)-(3-chloro-1,2,4-thiadiazol-5-yl)-oxyacetamide,
N-isopropyl-N-(4-chlorophenyl)-(3-chloro-1,2,4-thiadiazol-5-yl)-oxyacetamide,
N-isopropyl-N-(2,4-dichlorophenyl)-(3-chloro-1,2,4-thiadiazol-5-yl)-oxyacetamide and
N-isopropyl-N-(2-chlorophenyl)-(3-chloro-1,2,4-thiadiazol-5-yl)-oxyacetamide,
characterised in that heteroarenes of the general formula (II)

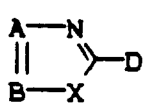

(II)

in which

A, B and X have the meanings given above in the case of formula (I) and

D represents a nucleofugic leaving group,

are reacted with N-isopropylhydroxyacetanilides of the general formula (III)

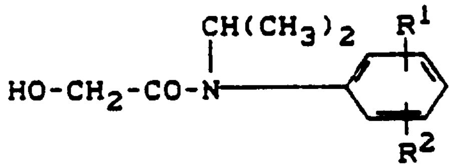

(III)

in which
$R^1$ and $R^2$ have the meanings given above in the case of formula (I)
if appropriate in the presence of a diluent, if appropriate in the presence of an acid-binding agent and if appropriate in the presence of a catalyst.

**2.** Process for the preparation of N-isopropylheteroaryloxyacetanilides of the formula (Ia)

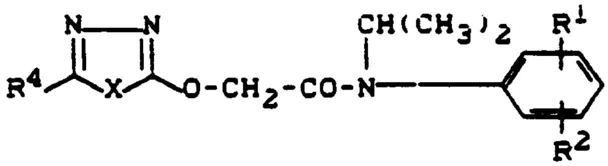

(Ia)

in which
- $R^1$ represents hydrogen, fluorine, chlorine, methyl, trifluoromethyl or methoxy,
- $R^2$ represents hydrogen,
- $R^4$ represents chlorine, trifluoromethyl, chlorodifluoromethyl, fluorodichloromethyl, dichloromethyl, trichloromethyl, pentafluoroethyl, heptafluoropropyl, methylthio, ethylthio, propylthio, methylsulphinyl, ethylsulphinyl, propylsulphinyl, methylsulphonyl, ethylsulphonyl, propylsulphonyl or phenyl and
- X represents oxygen or sulphur,

according to Claim 1, excluding the compound N-isopropyl-(5-trifluoromethyl-1,3,4-thiadiazol-2-yl)-oxyacetamide,
characterised in that heteroarenes of the general formula (II)

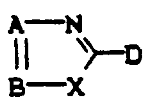

(II)

in which
A, B and X have the meanings given above in the case of formula (Ia) and
D represents a nucleofugic leaving group,
are reacted with N-isopropylhydroxyacetanilides of the general formula (III)

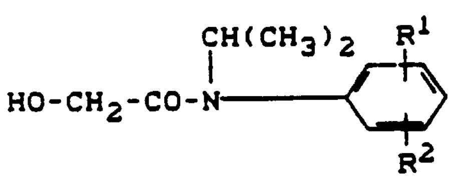

(III)

in which
$R^1$ and $R^2$ have the meanings given above in the case of formula (Ia)
if appropriate in the presence of a diluent, if appropriate in the presence of an acid-binding agent and if

appropriate in the presence of a catalyst.

**3.** Process for the preparation of N-isopropylheteroaryloxyacetanilides of the formula (Ib)

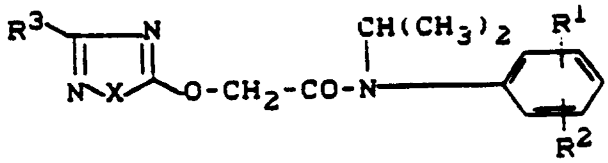

(Ib)

in which

- $R_1$ represents hydrogen, fluorine, chlorine, methyl, trifluoromethyl or methoxy,
- $R^2$ represents hydrogen,
- $R^3$ represents chlorine, difluoromethyl, trifluoromethyl, chlorodifluoromethyl, fluorodichloromethyl, dichloromethyl, methyl, ethyl, propyl, isopropyl, methylthio, ethylthio, propylthio, methylsulphonyl, ethylsulphonyl, propylsulphonyl, methylsulphinyl, ethylsulphinyl or propylsulphinyl and
- X represents oxygen or sulphur,

according to Claim 1, excluding the compounds N-isopropyl-(3-chloro-1,2,4-thiadiazol-5-yl)-oxyacetanilide,
N-isopropyl-N-(3-chlorophenyl)-(3-chloro-1,2,4-thiadiazol-5-yl)-oxyacetamide,
N-isopropyl-N-(4-chlorophenyl)-(3-chloro-1,2,4-thiadiazol-5-yl)-oxyacetamide, and
N-isopropyl-N-(2-chlorophenyl)-(3-chloro-1,2,4-thiadiazol-5-yl)-oxyacetamide,
characterised in that heteroarenes of the general formula (II)

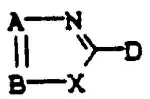

(II)

in which
A, B and X have the meanings given above in the case of formula (Ib) and
D represents a nucleofugic leaving group
are reacted with N-isopropylhydroxyacetanilides of the general formula (III)

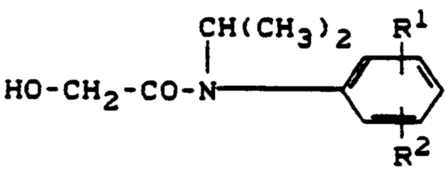

(III)

in which
$R^1$ and $R^2$ have the meanings given above in the case of the formula (Ib),
if appropriate in the presence of a diluent, if appropriate in the presence of an acid-binding agent and if appropriate in the presence of a catalyst.

**4.** Process for the preparation of N-isopropylheteroaryloxyacetanilides according to Claim 1 of the formula (Ic)

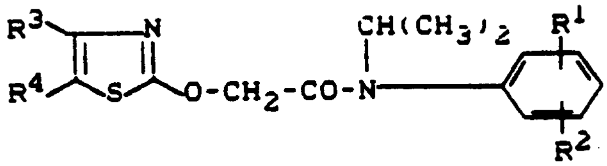

(Ic)

in which

$R^1$ represents hydrogen, fluorine, chlorine, methyl, trifluoromethyl or methoxy,

$R^2$ represents hydrogen,

$R^3$ represents chlorine, fluorine, methyl, difluoromethyl, dichloromethyl, chlorodifluoromethyl, fluorodichloromethyl, trichloromethyl or trifluoromethyl and

$R^4$ represents chlorine, cyano, difluoromethyl, dichloromethyl, chlorodifluoromethyl, fluorodichloromethyl, trichloromethyl or trifluoromethyl,

according to Claim 1,

characterised in that heteroarenes of the general formula (II)

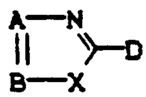

(II)

in which

A, B and X have the meanings given above in the case of formula (Ic) and

D represents a nucleofugic leaving group

are reacted with N-isopropylhydroxyacetamides of the general formula (III)

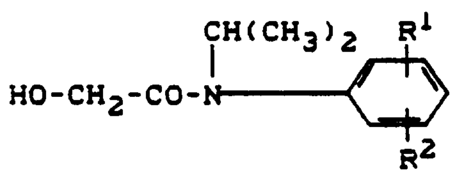

(III)

in which

$R^1$ and $R^2$ have the meanings given above in the case of formula (Ic)

if appropriate in the presence of a diluent, if appropriate in the presence of an acid-binding agent and if appropriate in the presence of a catalyst.

**5.** Process for the preparation of N-isopropyl-N-(4-fluorophenyl)-(5-trifluoromethyl-1,3,4-thiadiazol-5-yl)-oxyacetanilide of the formula

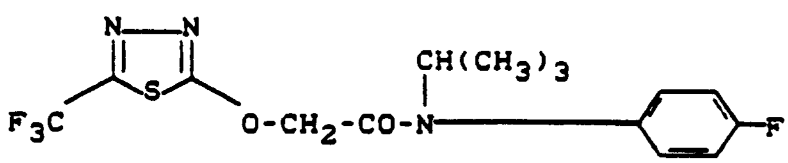

(33)

according to Claims 1 and 2, characterised in that a heteroarene of the general formula (II)

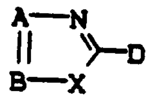

(II)

in which

A represents N,

B represents $F_3C$-C,

X represents S and

D represents a nucleofugic leaving group,

is reacted with an N-isopropylhydroxyacetanilide of the general formula (III)

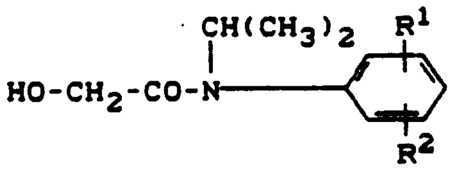

(III)

in which

$R^1$ represents 4-F and

$R^2$ represents H,

if appropriate in the presence of a diluent, if appropriate in the presence of an acid-binding agent and if appropriate in the presence of a catalyst.

**6.** Herbicidal agents, characterised in that they contain at least one N-isopropylheteroaryloxyacetanilide of the formula (I) according to Claim 1.

**7.** Method of combating undesired plant growth, characterised in that the N-isopropylheteroaryloxyacetanilides of the formula (I) according to Claim 1 are allowed to act on the plants or their environment.

**8.** Use of N-isopropylheteroaryloxyacetanilides of the formula (I) according to Claim 1 for combating undesired plant growth.

**9.** Process for the preparation of herbicidal agents, characterised in that N-isopropylheteroaryloxyacetanilides of the formula (I) according to Claim 1 are mixed with extenders and/or surface-active agents.

**Revendications**

**Revendications pour les Etats contractants suivants : BE, CH, DE, FR, GB, IT, LI, NL, SE**

**1.** N-isopropylanilides d'acides hétéroaryloxyacétiques de formule générale (I)

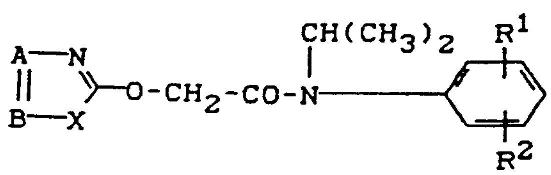

(I)

dans laquelle

A représente l'azote ou le groupement C-$R^3$, dans lequel

$R^3$ représente le fluor, le chlore, le brome, un groupe cyano, un groupe alkyle en $C_1$ à $C_4$

éventuellement substitué par du fluor et/ou du chlore, un groupe alkoxy en $C_1$ à $C_4$ éventuellement substitué par du fluor et/ou du chlore, un groupe alkylthio en $C_1$ à $C_4$ éventuellement substitué par du fluor et/ou du chlore, un groupe alkylsulfinyle en $C_1$ à $C_4$ ou un groupe alkylsulfonyle en $C_1$ à $C_4$,

B représente l'azote ou le groupement C-$R^4$, dans lequel,

$R^4$ est le fluor, le chlore, un groupe cyano, un groupe alkyle en $C_1$ à $C_4$ éventuellement substitué par du fluor et/ou du chlore, un groupe alkoxy en $C_1$ à $C_4$ éventuellement substitué par du fluor et/ou du chlore, un groupe alkylthio en $C_1$ à $C_4$ éventuellement substitué par du fluor et/ou du chlore, un groupe alkylsulfinyle en $C_1$ à $C_4$ éventuellement substitué par du fluor et/ou du chlore, un groupe alkylsulfonyle en $C_1$ à $C_4$ éventuellement substitué par du fluor et/ou du chlore, un groupe phényle éventuellement substitué par du fluor, du chlore, du brome, un radical alkyle en $C_1$ à $C_4$, trifluorométhyle, alkoxy en $C_1$ à $C_4$ ou trifluorométhoxy, ou le groupement -$CY_2$-Z-$R^5$, dans lequel

$R^5$ est un groupe alkyle en $C_1$ à $C_4$ éventuellement substitué par du fluor et/ou du chlore, un radical alkoxy en $C_1$ à $C_4$ ou un radical alkylthio en $C_1$ à $C_4$, ou représente un groupe phényle substitué le cas échéant par du fluor, du chlore, du brome, un radical alkyle en $C_1$ à $C_4$, trifluorométhyle, alkoxy en $C_1$ à $C_4$, trifluorométhoxy, alkylthio en $C_1$ à $C_4$ ou trifluorométhylthio,

Y est l'hydrogène, le fluor ou le chlore,

Z représente l'oxygène, le soufre, un groupe SO ou $SO_2$,

$R^1$ est l'hydrogène, le fluor, le chlore, le brome, un groupe cyano, nitro, un groupe alkyle en $C_1$ à $C_4$ éventuellement substitué par du fluor et/ou du chlore, un groupe alkoxy en $C_1$ à $C_4$ éventuellement substitué par du fluor et/ou du chlore ou un groupe alkylthio en $C_1$ à $C_4$ éventuellement substitué par du fluor et/ou du chlore,

$R^2$ est l'hydrogène, le fluor, le chlore, le brome, un groupe méthyle, trifluorométhyle ou méthoxy et

X est l'oxygène ou le soufre,

excepté les composés N-isopropylanilide d'acide (5-trifluorométhyl-1,3,4-thiadiazole-2-yl)-oxyacétique,
N-isopropylanilide d'acide (3-trichlorométhyl-1,2,4-thiadiazole-5-yl)oxyacétique,
N-isopropylanilide d'acide (3-chloro-1,2,4-thiadiazole-5-yl)oxyacétique,
N-isopropyl-N-(3-chlorophényl)amide d'acide (3-chloro-1,2,4-thiadiazole-5-yl)oxyacétique,
N-isopropyl-N-(4-chlorophényl)amide d'acide (3-chloro-1,2,4-thiadiazole-5-yl)oxyacétique,
N-isopropyl-N-(2,4-dichlorophényl)amide d'acide (3-chloro-1,2,4-thiadiazole-5-yl)oxyacétique et
N-isopropyl-N-(2-chlorophényl)amide d'acide (3-chloro-1,2,4-thiadiazole-5-yl)oxyacétique.

2. N-isopropylanilides d'acides hétéroaryloxyacétiques de formule (Ia)

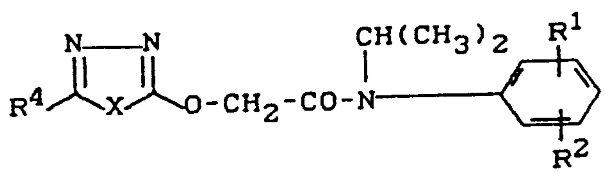

(Ia)

dans laquelle

$R^1$ est l'hydrogène, le fluor, le chlore, un groupe méthyle, trifluorométhyle ou méthoxy,

$R^2$ est l'hydrogène,

$R^4$ est le chlore, un groupe trifluorométhyle, chlorodifluorométhyle, fluorodichlorométhyle, dichlorométhyle, trichlorométhyle, pentafluoréthyle, heptafluoropropyle, méthylthio, éthylthio, propylthio, méthylsulfinyle, éthylsulfinyle, propylsulfinyle, méthylsulfonyle, éthylsulfonyle, propylsulfonyle ou phényle et

X est l'oxygène ou le soufre,

suivant la revendication 1, excepté le composé N-isopropylanilide d'acide (5-trifluorométhyl-1,3,4-thiadiazole-2-yl)oxyacétique.

3. N-isopropylanilides d'acides hétéroaryloxyacétiques de formule (Ib)

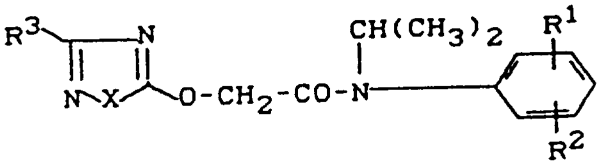

(Ib)

dans laquelle

- $R^1$ est l'hydrogène, le fluor, le chlore, un groupe méthyle, trifluorométhyle ou méthoxy,
- $R^2$ est l'hydrogène,
- $R^3$ est le chlore, un groupe difluorométhyle, trifluorométhyle, chlorodifluorométhyle, fluorodichlorométhyle, dichlorométhyle, méthyle, éthyle, propyle, isopropyle, méthylthio, éthylthio, propylthio, méthylsulfonyle, éthylsulfonyle, propylsulfonyle, méthylsulfinyle, éthylsulfinyle ou propylsulfinyle, et
- X est l'oxygène ou le soufre,

suivant la revendication 1, excepté les composés
N-isopropylanilide d'acide (3-chloro-1,2,4-thiadiazole-5-yl)oxyacétique,
N-isopropyl-N-(3-chlorophényl)amide d'acide (3-chloro-1,2,4-thiadiazole-5-yl)oxyacétique,
N-isopropyl-N-(4-chlorophényl)amide d'acide (3-chloro-1,2,4-thiadiazole-5-yl)oxyacétique, et
N-isopropyl-N-(2-chlorophényl)amide d'acide (3-chloro-1,2,4-thiadiazole-5-yl)oxyacétique.

4. N-isopropylanilides d'acides hétéroaryloxyacétiques de formule (Ic)

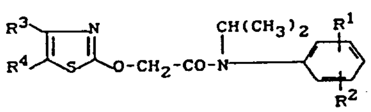

(Ic)

dans laquelle

- $R^1$ représente l'hydrogène, le fluor, le chlore, un groupe méthyle, trifluorométhyle ou méthoxy,
- $R^2$ est l'hydrogène,
- $R^3$ représente le chlore, le fluor, un groupe méthyle, difluorométhyle, dichlorométhyle, chlorodifluorométhyle, fluorodichlorométhyle, trichlorométhyle ou trifluorométhyle et
- $R^4$ est le chlore, un groupe cyano, difluorométhyle, dichlorométhyle, chlorodifluorométhyle, fluorodichlorométhyle, trichlorométhyle ou trifluorométhyle,

suivant la revendication 1.

5. N-isopropyl-N-(4-fluorophényl)amide d'acide (5-trifluorométhyl-1,3,4-thiadiazole-5-yl)oxyacétique de formule

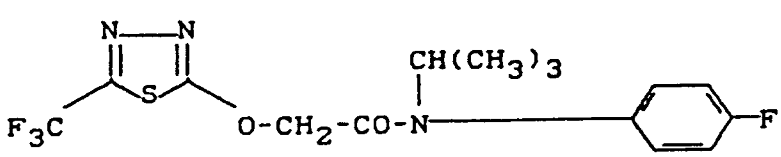

(33)

suivant les revendications 1 et 2.

6. Procédé de production de N-isopropylanilides d'acides hétéroaryloxyacétiques de formule générale (I) suivant la revendication 1, caractérisé en ce qu'on fait réagir des hétéroarènes de formule générale (II)

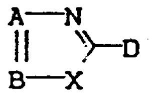

(II)

dans laquelle

A, B et X ont les définitions indiquées dans la revendication 1 et

D représente un groupe partant nucléofuge,

avec des N-isopropylanilides d'acide hydroxyacétique de formule générale (III)

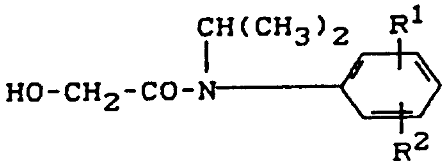

(III)

dans laquelle

$R^1$ et $R^2$ ont les définitions indiquées dans la revendication 1,

le cas échéant en présence d'un diluant, en la présence éventuelle d'un accepteur d'acide et, le cas échéant, en présence d'un catalyseur.

**7.** Compositions herbicides caractérisées par une teneur en au moins un N-isopropylanilide d'acide hétéroaryloxyacétique de formule (I) suivant la revendication 1.

**8.** Procédé pour combattre la croissance de plantes indésirables, caractérisé en ce qu'on fait agir sur les plantes ou sur leur milieu des N-isopropylanilides d'acides hétéroaryloxyacétiques de formule (I) suivant la revendication 1.

**9.** Utilisation de N-isopropylanilides d'acides hétéroaryloxyacétiques de formule (I) suivant la revendication 1 pour combattre la croissance de plantes indésirables.

**10.** Procédé de préparation de compositions herbicides, caractérisé en ce qu'on mélange des N-isopropylanilides d'acides hétéroaryloxyacétiques de formule (I) suivant la revendication 1 avec des diluants et/ou des agents tensio-actifs.

**Revendications pour l'Etat contractant suivant : ES**

**1.** Procédé de production de N-isopropylanilides d'acides hétéroaryloxyacétiques de formule générale (I)

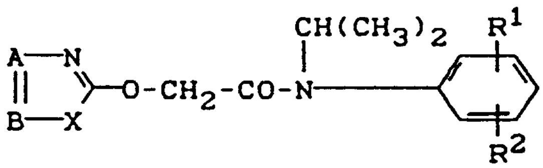

(I)

dans laquelle

A représente l'azote ou le groupement C-$R^3$, dans lequel

$R^3$ représente le fluor, le chlore, le brome, un groupe cyano, un groupe alkyle en $C_1$ à $C_4$ éventuellement substitué par du fluor et/ou du chlore, un groupe alkoxy en $C_1$ à $C_4$ éventuellement substitué par du fluor et/ou du chlore, un groupe alkylthio en $C_1$ à $C_4$ éventuellement substitué par du fluor et/ou du chlore, un groupe alkylsulfinyle en $C_1$ à $C_4$ ou un groupe alkylsulfonyle en $C_1$ à $C_4$,

B représente l'azote ou le groupement C-$R^4$, dans lequel,

$R^4$ est le fluor, le chlore, un groupe cyano, un groupe alkyle en $C_1$ à $C_4$ éventuellement substitué par du fluor et/ou du chlore, un groupe alkoxy en $C_1$ à $C_4$ éventuellement substitué par du fluor et/ou du chlore, un groupe alkylthio en $C_1$ à $C_4$ éventuellement substitué par du fluor et/ou du chlore, un groupe alkylsulfinyle en $C_1$ à $C_4$ éventuellement substitué par du fluor et/ou du chlore, un groupe alkylsulfonyle en $C_1$ à $C_4$ éventuellement substitué par du fluor et/ou du chlore, un groupe phényle éventuellement substitué par du fluor, du chlore, du brome, un radical alkyle en $C_1$ à $C_4$, trifluorométhyle, alkoxy en $C_1$ à $C_4$ ou trifluorométhoxy, ou le groupement $-CY_2-Z-R^5$, dans lequel

$R^5$ est un groupe alkyle en $C_1$ à $C_4$ éventuellement substitué par du fluor et/ou du chlore, un radical alkoxy en $C_1$ à $C_4$ ou un radical alkylthio en $C_1$ à $C_4$, ou représente un groupe phényle substitué le cas échéant par du fluor, du chlore, du brome, un radical alkyle en $C_1$ à $C_4$, trifluorométhyle, alkoxy en $C_1$ à $C_4$, trifluorométhoxy, alkylthio en $C_1$ à $C_4$ ou trifluorométhylthio,

Y est l'hydrogène, le fluor ou le chlore,

Z représente l'oxygène, le soufre, un groupe SO ou $SO_2$,

$R^1$ est l'hydrogène, le fluor, le chlore, le brome, un groupe cyano, nitro, un groupe alkyle en $C_1$ à $C_4$ éventuellement substitué par du fluor et/ou du chlore, un groupe alkoxy en $C_1$ à $C_4$ éventuellement substitué par du fluor et/ou du chlore ou un groupe alkylthio en $C_1$ à $C_4$ éventuellement substitué par du fluor et/ou du chlore,

$R^2$ est l'hydrogène, le fluor, le chlore, le brome, un groupe méthyle, trifluorométhyle ou méthoxy et

X est l'oxygène ou le soufre,

excepté les composés N-isopropylanilide d'acide (5-trifluorométhyl-1,3,4-thiadiazole-2-yl)oxyacétique, N-isopropylanilide d'acide (3-trichlorométhyl-1,2,4-thiadiazole-5-yl)oxyacétique, N-isopropylanilide d'acide (3-chloro-1,2,4-thiadiazole-5-yl)oxyacétique, N-isopropyl-N-(3-chlorophényl)amide d'acide (3-chloro-1,2,4-thiadiazole-5-yl)oxyacétique, N-isopropyl-N-(4-chlorophényl)amide d'acide (3-chloro-1,2,4-thiadiazole-5-yl)oxyacétique, N-isopropyl-N-(2,4-dichlorophényl)amide d'acide (3-chloro-1,2,4-thiadiazole-5-yl)oxyacétique et N-isopropyl-N-(2-chlorophényl)amide d'acide (3-chloro-1,2,4-thiadiazole-5-yl)oxyacétique, caractérisé en ce qu'on fait réagir des hétéroarènes de formule générale (II)

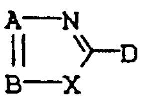

(II)

dans laquelle

A, B et X ont les définitions indiquées ci-dessus pour la formule (I) et

D est un groupe partant nucléofuge,

avec des N-isopropylanilides d'acide hydroxyacétique de formule (III)

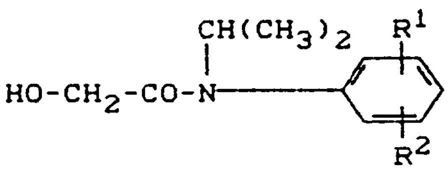

(III)

dans laquelle

$R^1$ et $R^2$ ont les définitions indiquées ci-dessus pour la formule (I),

le cas échéant en présence d'un diluant, en la présence éventuelle d'un accepteur d'acide et le cas échéant en présence d'un catalyseur.

**2.** Procédé de production de N-isopropylanilides d'acides hétéroaryloxyacétiques de formule (Ia)

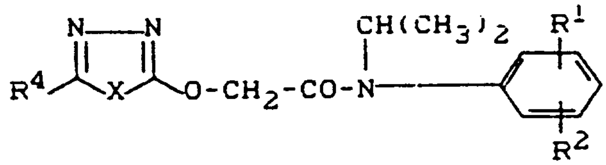

(Ia)

dans laquelle

- $R^1$ est l'hydrogène, le fluor, le chlore, un groupe méthyle, trifluorométhyle ou méthoxy,
- $R^2$ est l'hydrogène,
- $R^4$ est le chlore, un groupe trifluorométhyle, chlorodifluorométhyle, fluorodichlorométhyle, dichlorométhyle, trichlorométhyle, pentafluoréthyle, heptafluoropropyle, méthylthio, éthylthio, propylthio, méthylsulfinyle, éthylsulfinyle, propylsulfinyle, méthylsulfonyle, éthylsulfonyle, propylsulfonyle ou phényle et
- X est l'oxygène ou le soufre,

suivant la revendication 1, excepté le composé N-isopropylanilide d'acide (5-trifluorométhyl-1,3,4-thiadiazole-2-yl)oxyacétique,
caractérisé en ce qu'on fait réagir des hétéroarènes de formule générale (II)

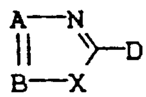

(II)

dans laquelle

- A, B et X ont les définitions indiquées ci-dessus pour la formule (Ia) et
- D est un groupe partant nucléofuge,

avec des N-isopropylanilides d'acide hydroxyacétique de formule générale (III)

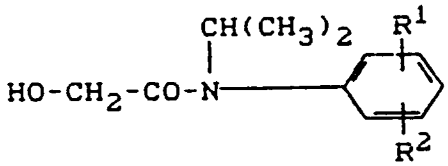

(III)

dans laquelle

- $R^1$ et $R^2$ ont les définitions indiquées ci-dessus pour la formule (Ia),

le cas échéant en présence d'un diluant, en la présence éventuelle d'un accepteur d'acide et, le cas échéant, en présence d'un catalyseur.

**3.** Procédé de production de N-isopropylanilides d'acides hétéroaryloxyacétiques de formule (Ib)

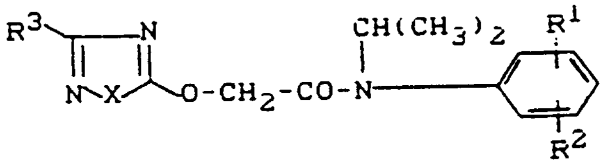

(Ib)

dans laquelle

- $R^1$ est l'hydrogène, le fluor, le chlore, un groupe méthyle, trifluorométhyle ou méthoxy,
- $R^2$ est l'hydrogène,
- $R^3$ est le chlore, un groupe difluorométhyle, trifluorométhyle, chlorodifluorométhyle, fluorodichlorométhyle, dichlorométhyle, méthyle, éthyle, propyle, isopropyle, méthylthio, éthylthio, propylthio, méthylsulfonyle, éthylsulfonyle, propylsulfonyle, méthylsulfinyle, éthylsulfinyle ou propylsulfinyle et
- X est l'oxygène ou le soufre,

suivant la revendication 1, excepté les composés
N-isopropylanilide d'acide (3-chloro-1,2,4-thiadiazole-5-yl)oxyacétique,
N-isopropyl-N-(3-chlorophényl)amide d'acide (3-chloro-1,2,4-thiadiazole-5-yl)oxyacétique,
N-isopropyl-N-(4-chlorophényl)amide d'acide (3-chloro-1,2,4-thiadiazole-5-yl)oxyacétique, et
N-isopropyl-N-(2-chlorophényl)amide d'acide (3-chloro-1,2,4-thiadiazole-5-yl)oxyacétique,
caractérisé en ce qu'on fait réagir des hétéroarènes de formule générale (II),

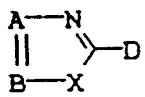
(II)

dans laquelle

- A, B et X ont les définitions indiquées ci-dessus pour la formule (Ib) et
- D est un groupe partant nucléofuge,

avec des N-isopropylanilides d'acide hydroxyacétique de formule générale (III),

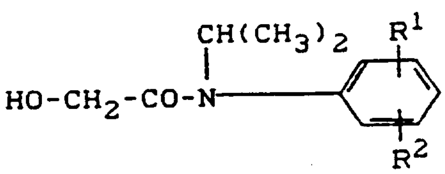
(III)

dans laquelle

- $R^1$ et $R^2$ ont les définitions indiquées ci-dessus pour la formule (Ib),

le cas échéant en présence d'un diluant, en la présence éventuelle d'un accepteur d'acide et le cas échéant en présence d'un catalyseur.

**4.** Procédé de production de N-isopropylanilides d'acides hétéroaryloxyacétiques de formule (Ic)

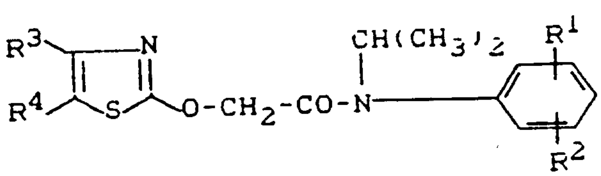
(Ic)

dans laquelle

- $R^1$ représente l'hydrogène, le fluor, le chlore, un groupe méthyle, trifluorométhyle ou méthoxy,
- $R^2$ est l'hydrogène,
- $R^3$ représente le chlore, le fluor, un groupe méthyle, difluorométhyle, dichlorométhyle, chlorodifluorométhyle, fluorodichlorométhyle, trichlorométhyle ou trifluorométhyle et
- $R^4$ est le chlore, un groupe cyano, difluorométhyle, dichlorométhyle, chlorodifluorométhyle, fluorodichlorométhyle, trichlorométhyle ou trifluorométhyle,

suivant la revendication 1,
caractérisé en ce qu'on fait réagir des hétéroarènes de formule générale (II)

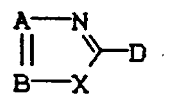

(II)

dans laquelle

A, B et X ont les définitions indiquées ci-dessus pour la formule (Ic) et

D est un groupe partant nucléofuge,

avec des N-isopropylanilides d'acide hydroxyacétique de formule générale (III),

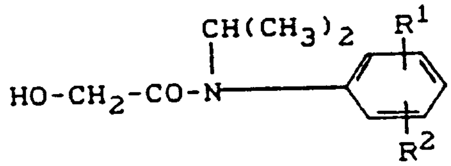

(III)

dans laquelle

$R^1$ et $R^2$ ont les définitions indiquées ci-dessus pour la formule (Ic),

le cas échéant en présence d'un diluant, en la présence éventuelle d'un accepteur d'acide et le cas échéant en présence d'un catalyseur.

**5.** Procédé de production de N-isopropyl-N-(4-fluorophényl)amide d'acide (5-trifluorométhyl-1,3,4-thiadiazole-5-yl)oxyacétique de formule

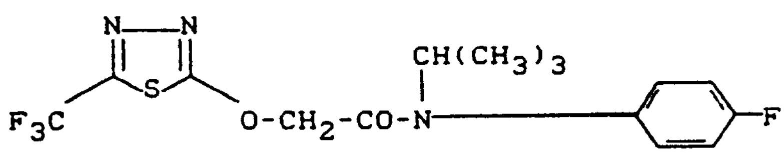

(33)

suivant les revendications 1 et 2, caractérisé en ce qu'on fait réagir un hétéroarène de formule générale (II)

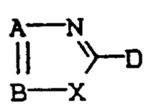

(II)

dans laquelle

A représente N,

B est un groupe $F_3C$-C,

X représente S et

D est un groupe partant nucléofuge,

avec un N-isopropylanilide d'acide hydroxyacétique de formule générale (III)

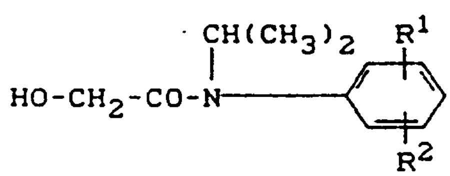

(III)

dans laquelle

$R^1$ représente 4-F et

$R^2$ représente H,

le cas échéant en présence d'un diluant, en la présence éventuelle d'un accepteur d'acide et, le cas échéant, en présence d'un catalyseur.

**6.** Compositions herbicides, caractérisées par une teneur en au moins un N-isopropylanilide d'acide hétéroaryloxyacétique de formule (I) suivant la revendication 1.

**7.** Procédé pour combattre la croissance de plantes indésirables, caractérisé en ce qu'on fait agir sur les plantes ou sur leur milieu des N-isopropylanilides d'acides hétéroaryloxyacétiques de formule (I) suivant la revendication 1.

**8.** Utilisation de N-isopropylanilides d'acides hétéroaryloxyacétiques de formule (I) suivant la revendication 1 pour combattre la croissance de plantes indésirables.

**9.** Procédé de préparation de compositions herbicides, caractérisé en ce qu'on mélange des N-isopropylanilides d'acides hétéroaryloxyacétiques de formule (I) suivant la revendication 1 avec des diluants et/ou des agents tensio-actifs.